# EUROPEAN PATENT SPECIFICATION

(11) **EP 2 872 653 B1**
(45) Date of publication and mention of the grant of the patent: **13.03.2019**
(21) Application number: 13817114.5
(22) Date of filing: 12.07.2013
(51) Int. Cl.: C12Q 1/6874, C12Q 1/6881, C12Q 1/6888, A61K 39/00, A61K 35/17, G06F 19/18

(54) **PERSONALIZED CANCER VACCINES AND ADOPTIVE IMMUNE CELL THERAPIES**
PERSONALISIERTE KREBSIMPFSTOFFE UND ADAPTIVE IMMUNZELLTHERAPIEN
VACCINS ANTICANCÉREUX PERSONNALISÉS ET THÉRAPIES CELLULAIRES IMMUNITAIRES ADOPTIVES

(30) Priority: 12.07.2012 US 201261670931 P
(43) Date of publication of application: 20.05.2015
(60) Divisional application: 19155189.4
(73) Proprietor: Persimmune, Inc., San Diego, CA 92121 (US)
(72) Inventor: VITIELLO, Maria, Antonia, La Jolla, CA 92037 (US)
(74) Representative: Gulde & Partner
(86) International application number: PCT/US2013/050362
(87) International publication number: WO 2014/012051

(56) References cited:
- US-A1- 2002 085 997
- US-A1- 2006 134 129
- US-A1- 2011 177 079
- US-A1- 2011 293 637
- US-A1- 2011 293 637
- CORNELIS J.M. MELIEF ET AL: "Immunotherapy of established (pre)malignant disease by synthetic long peptide vaccines", NATURE REVIEWS CANCER, vol. 8, no. 5, 1 May 2008 (2008-05-01), pages 351-360, XP055019108, ISSN: 1474-175X, DOI: 10.1038/nrc2373
- J. C. CASTLE ET AL: "Exploiting the mutanome for tumor vaccination", CANCER RESEARCH, 1 January 2012 (2012-01-01), XP055018897, ISSN: 0008-5472, DOI: 10.1158/0008-5472.CAN-11-3722
- KARL ET AL.: 'Cost-effective sequence-based nonhuman primate MHC class I genotyping from RNA.' METHODS vol. 49, no. 1, September 2009, pages 11 - 7, XP026501218
- MATERA: 'The choice of the antigen in the dendritic cell-based vaccine therapy for prostate cancer.' CANCER TREAT REV. vol. 36, no. 2, April 2010, pages 131 - 41, XP026942926
- SIDNEY ET AL.: 'HLA class I supertypes: a revised and updated classification.' BMC IMMUNOL vol. 99, 22 January 2008, page 1, XP021033200
- H-G Rammensee ET AL: "Cancer Vaccines: Some Basic Considerations" In: "Genomic and Personalized Medicine", 11 November 2008 (2008-11-11), Elsevier, XP55348899, page 573589, DOI: 10.1016/B978-0-12-369420-1.00050-0,

## Description

### FIELD OF THE INVENTION

This invention relates to the identification of mutations in expressed genes of cancer cells from cancer patients and use of the mutations to prepare cancer vaccines and adoptive immune cell therapies.

### BACKGROUND OF THE INVENTION

Cancer is the second leading cause of death in the US. The estimates for 2010 are that approximately 570,000 people will die from cancer and 1.5 million new cases will be diagnosed (1). For early stage cancers (those that have not spread to the lymph nodes and are non-metastatic) surgical removal is a very effective treatment. However, for more advanced cases, standard, non-specific cancer treatments (chemo and radiotherapy) are used. These treatments affect many healthy cells and result in elevated toxicity. One of the most important principles of medical ethics, "primum non nocere" (first do no harm), is often not applicable in the treatment of cancer, where patients are submitted to very toxic therapeutic protocols that are effective in only a percentage of treated individuals. Moreover, even individuals that initially are treated successfully are at risk for relapses, and become more difficult to treat for each succeeding relapse.

The idea of employing the adaptive immune system to kill cancer cells without harming normal cells has been a goal for many decades (for review see Dunn 2002 (2)). To become a cancer cell, a healthy cell undergoes multiple somatic mutations (3, 4). Such mutations may be targets of the adaptive immune system, which performs the function of recognizing and eliminating small variations from self. The possibility of using immunotherapy for successfully treating cancers is gaining support due to findings that (a) tumor-specific lymphocytes can be isolated from patients with tumors (6, 7); (b) the presence of tumor-specific lymphocytes infiltrating the tumor (or in circulation) correlate with good prognosis (8); and (c) antigens recognized by T lymphocytes on the tumor cell have been identified (9, 10, 12). Also related are (a) the demonstrated effectiveness of adoptive cellular immunotherapy for cytomegalovirus (CMV) infection and lymphomas associated with the Epstein Barr virus (EBV) in patients that underwent bone marrow transplantation (BMT) (11), and (b) the success of adoptive cell therapy in the treatment of patients with metastatic melanomas (7).

Basic considerations relating to the provision of cancer vaccines are summarized in Rammensee et al; "Cancer Vaccines: Some Basic Considerations", Genomic and Personalized Medicine, 2009, 573-589.

However, tumor antigen identification and its translation to immunotherapy still face many problems. Therefore, being able to define antigens in an easier and more efficient manner is an advantage. The process of identifying and utilizing antigens, as described herein, allows for the individualized diagnosis and treatment of patients which increase the likelihood of treatment success.

### SUMMARY OF THE INVENTION

The present invention is directed to a method of manufacturing a cancer specific T lymphocytes (CTL) cell line for use in treatment of a cancer patient as defined in the claims. Provided herein are methods to identify mutations in expressed genes of cancer cells from cancer patients and to use the mutations to prepare cancer vaccines and adoptive immune cell therapies for treating the cancer patients. Nucleic acid sequences from the cancer cells are obtained by a parallel sequencing platform, which employs parallel processing of the nucleic acid of cancer cells leading to sequence reads and mapping of the sequence reads to a database with reference gene sequences. In some embodiments, the parallel sequencing platform employs certain filtering of the sequencing results such as a depth of coverage less than 20 x and/or by not filtering with a base alignment quality (BAQ) algorithm.

Mutant sequences which code for all or a portion of an expressed gene are identified as those which have a mutant position amino acid which substitutes for a wildtype position amino acid located at the same position in the wildtype sequence of the protein.

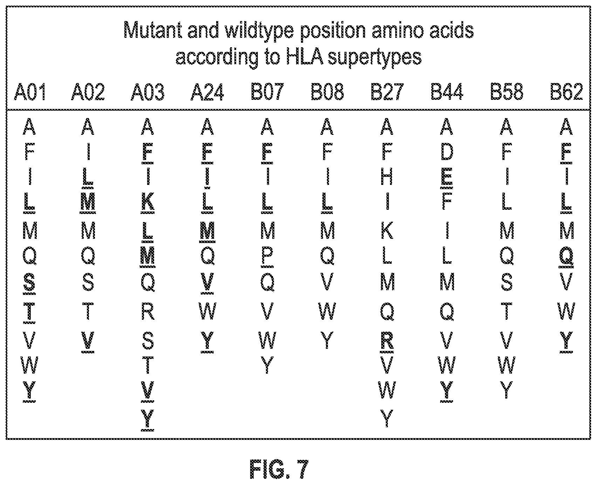

A further selection of mutant sequences can be achieved by identifying an HLA class and/or HLA supertype of the cancer patient and then selecting one or more amino acids for the particular HLA class and/or HLA supertype as the mutant position amino acid and/or wildtype position amino acid. Candidate mutant position amino acid and/or wildtype position amino acid for each HLA class and/or HLA supertype are shown in FIG. 7. Alternatively, one can ignore the HLA class and/or HLA supertype of the individual and make a further selection using one or more amino acids selected from the group consisting of tyrosine, phenylalanine, leucine, isoleucine, methionine, valine and alanine.

In accordance with the invention, peptides containing mutant sequences of interest are evaluated for their ability to bind to HLA histocompatibility antigens of the cancer patient. This can be carried out in silico using computer-based algorithm(s) for predicting HLA binding peptides. Alternatively, or in addition, the ability to bind to HLA histocompatibility antigens is carried out by synthesizing the peptides and testing them for binding to HLA histocompatibility antigens. The testing of sequences for binding to HLA histocompatibility antigens is not a requirement but may be used to narrow the set of potential cancer antigens prior to further testing.

In further embodiments, peptides containing the mutant sequences of interest may be synthesized and evaluated for activating cytotoxic T lymphocytes (CTL) cell lines prepared from the cancer patient or from an HLA-matched donor (matched for class and/or supertype). In such cases, the CTL cell lines are obtained by contacting two cell types: mononuclear cells from the cancer patient or from the HLA-matched donor and cancer cells from the cancer patient. The CTL cell lines may be prepared using mononuclear cells that are enriched in CD8⁺ cells and may further include the addition of autologous CD4⁺ T cells and/or dendritic cells from the cancer patient or autologous CD4⁺ T cells and/or dendritic cells from the HLA-matched donor. In embodiments in which only the peptides are used to stimulate the CTLs, donor matching on the HLA class and/or subtype is all that is required.

In another embodiment, methods to identify mutations in expressed genes of cancer cells from cancer patients and to use the mutations to prepare cancer vaccines and adoptive immune cell therapies for treating the cancer patients involves obtaining nucleic acid sequences from the cancer cells by the parallel sequencing platform as discussed. Mutant sequences which code for all or a portion of an expressed gene are identified as those which have a mutant position amino acid which substitutes for a wildtype position amino acid located at the same position in the wildtype sequence of the protein. Peptides containing the mutant sequences of interest and optionally the corresponding wildtype sequence peptides are synthesized and evaluated for activating cytotoxic T lymphocytes (CTL) cell lines prepared from the cancer patient or from an HLA-matched donor. In such cases, the CTL cell lines are obtained by contacting mononuclear cells from the cancer patient or from the HLA-matched donor with cancer cells from the cancer patient. The CTL cell lines may be prepared using mononuclear cells that are enriched in CD8⁺ cells and may further include the addition of autologous CD4⁺ T cells and/or dendritic cells from the cancer patient or autologous CD4⁺ T cells and/or dendritic cells from the HLA-matched donor.

Specifically, in one aspect, it is disclosed a method of identifying cancer antigens for preparing a cancer vaccine, comprising
a) obtaining a plurality of mutant sequences from the nucleic acid of cancer cells from a cancer patient, said mutant sequences coding for all or a portion of an expressed gene and wherein the mutant sequences each have a mutant position amino acid which substitutes for a wildtype position amino acid, or other mutation (e.g., insertion or deletion), located at the same position in the wildtype sequence of the protein, wherein said mutant sequences are obtained using a parallel sequencing platform, said parallel sequencing platform employing parallel processing of said nucleic acid of cancer cells leading to sequence reads and mapping of the sequence reads to a database with reference gene sequences; and
b) selecting mutant sequences from those identified in step a) by identifying an HLA class or supertype of the cancer patient and then selecting an amino acid for said HLA class or supertype as the mutant position amino acid and/or wildtype position amino acid using FIG. 7, wherein cancer antigens for preparing a cancer vaccine are identified.

In another aspect it is disclosed a method of identifying cancer antigens for preparing a cancer vaccine, comprising
a) obtaining a plurality of mutant sequences from the nucleic acid of cancer cells from a cancer patient, said mutant sequences coding for all or a portion of an expressed gene and wherein the mutant sequences each have a mutant position amino acid which substitutes for a wildtype position amino acid, or other mutation (e.g., insertion or deletion), located at the same position in the wildtype sequence of the protein, wherein said mutant sequences are obtained using a parallel sequencing platform, said parallel sequencing platform employing parallel processing of said nucleic acid of cancer cells leading to sequence reads and mapping of the sequence reads to a database with reference gene sequences; and
b) identifying at least one mutant sequence for preparing a cancer vaccine from the plurality of mutant sequences obtained in step a) by determining that at least one peptide encoded by the at least one mutant sequence binds to an HLA class or supertype of the cancer patient.

In another aspect, it is disclosed a method for predicting the effectiveness of an therapy described herein (e.g., adoptive transfer and vaccination) by (a) identifying at least one mutant nucleic acid sequence from the patient (e.g., from the cancer of the patient), wherein the mutant sequence codes for all or a portion of an expressed gene and wherein the encoded protein or peptide comprises a mutant amino acid substitution or other mutation relative to the wildtype, and (b) determining the binding capacity of the mutant peptide with the HLA class or supertype of the patient, wherein the strength, amount, and/or capacity for HLA binding is indicative of the patient's likely responsiveness to therapy, wherein greater binding indicates higher responsiveness. The binding capacity may be determined either using in silico techniques, such as the ones described herein, or by in vitro testing in which the identified mutant peptide is assess for binding to any one or more of the patient's HLA-expressing cells, as described herein, or to another cell expressing the same HLA class or supertype as the patient.

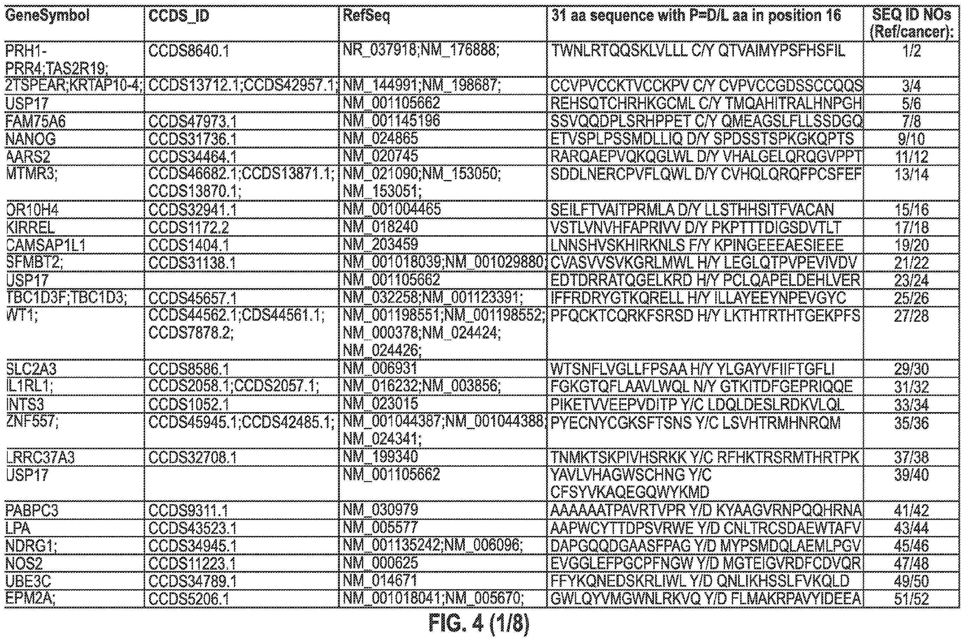

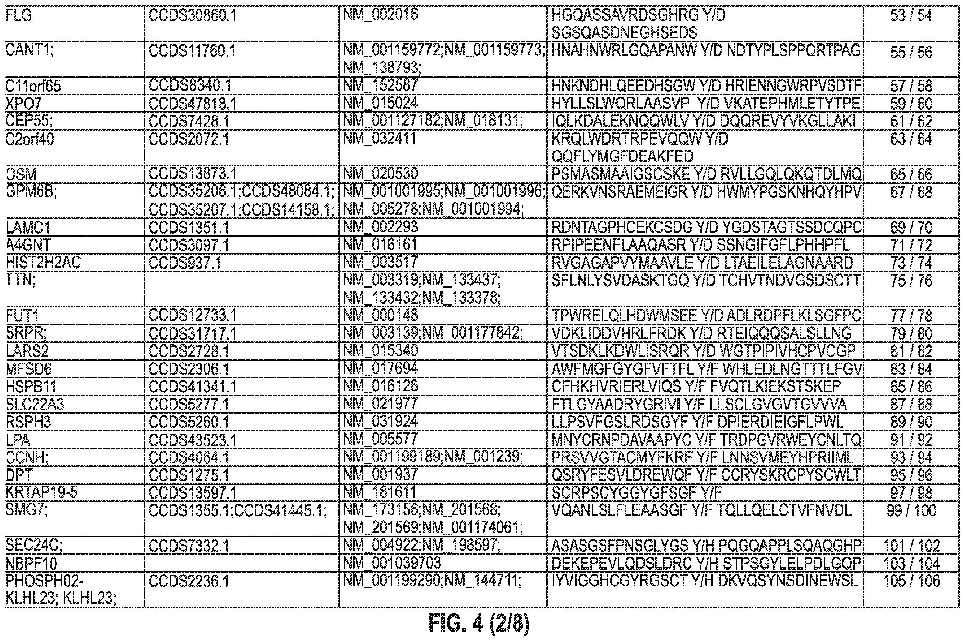

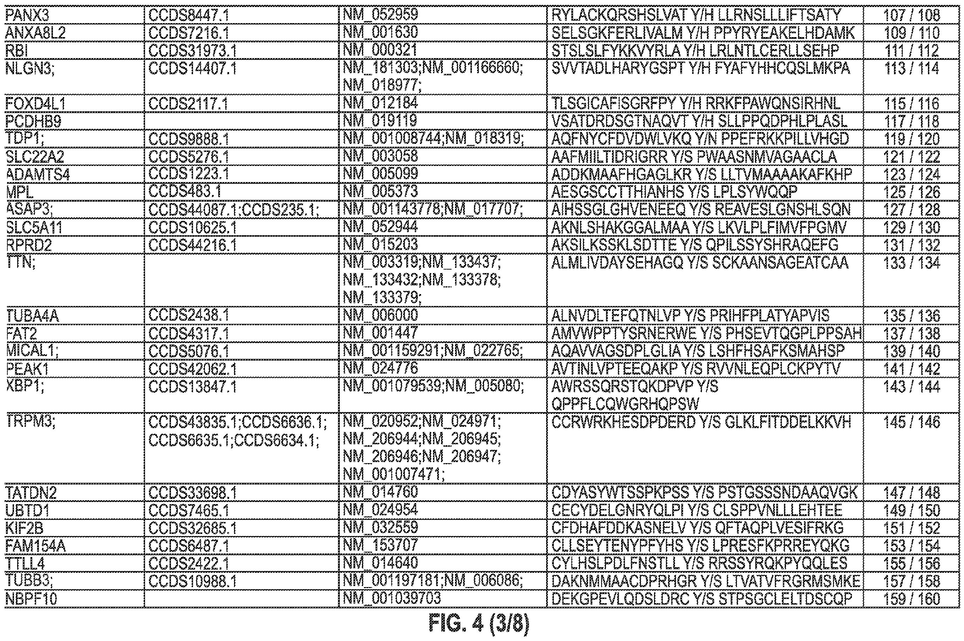

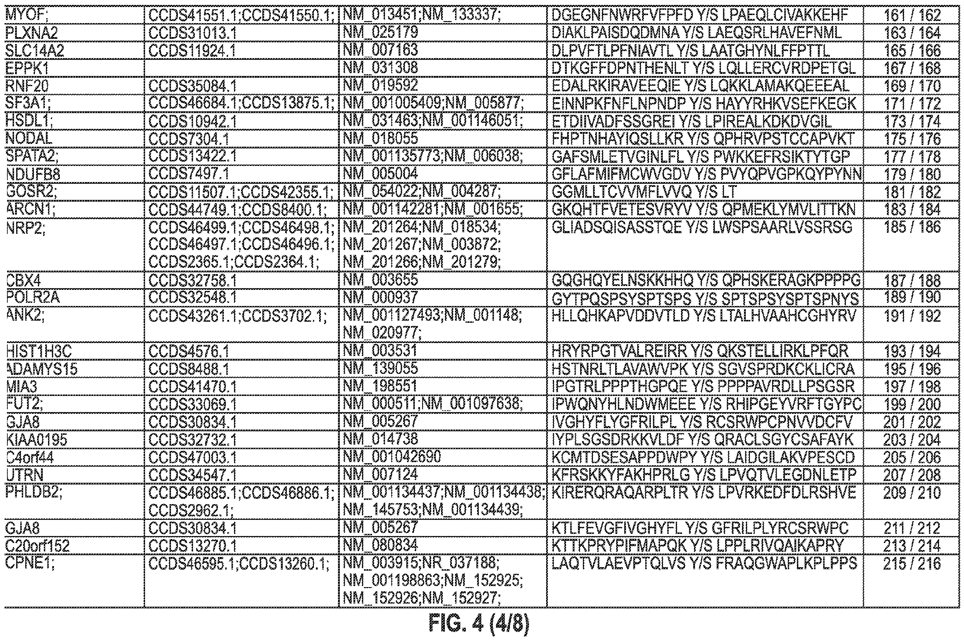

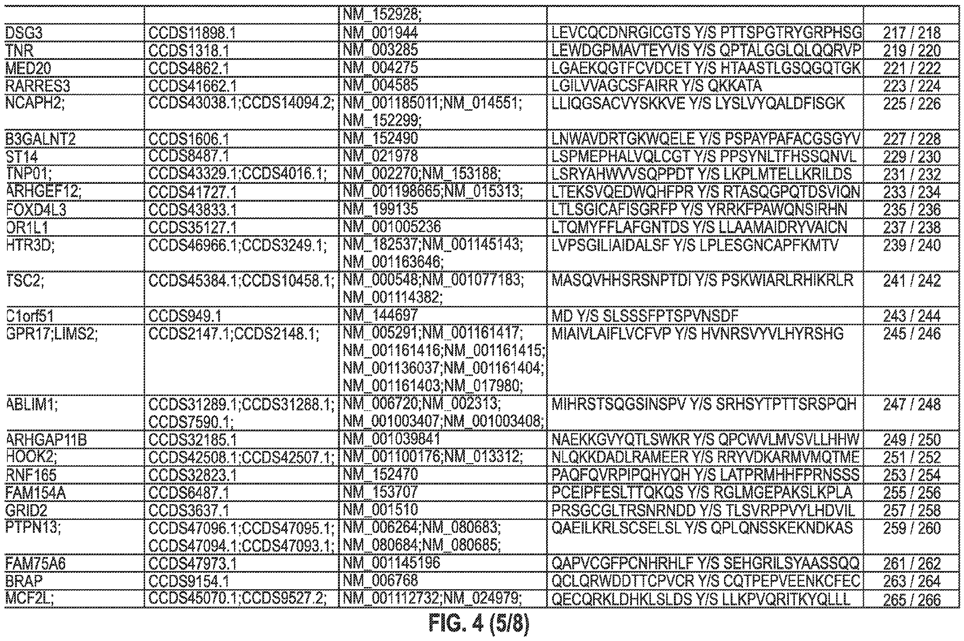

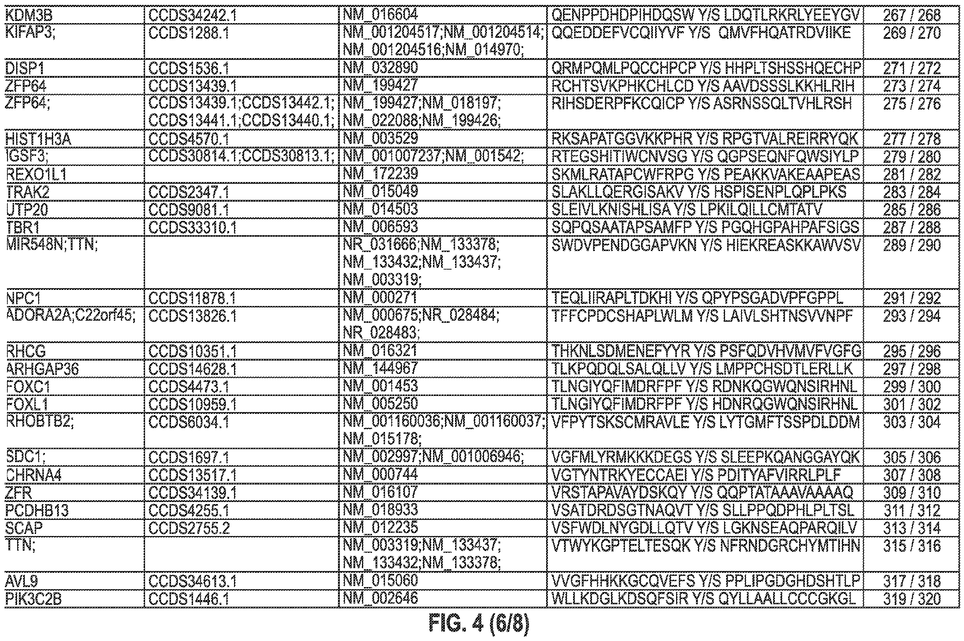

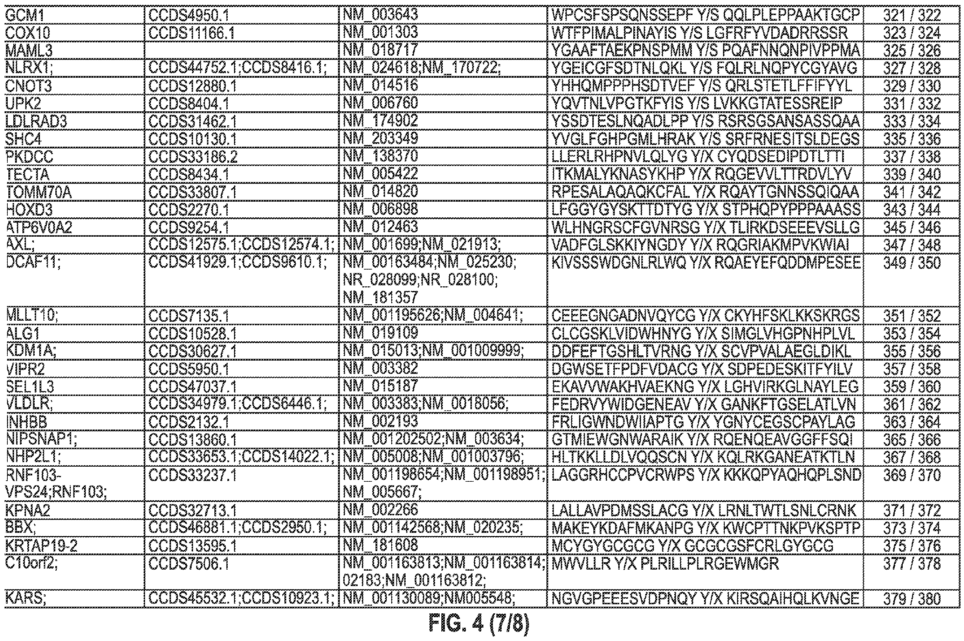

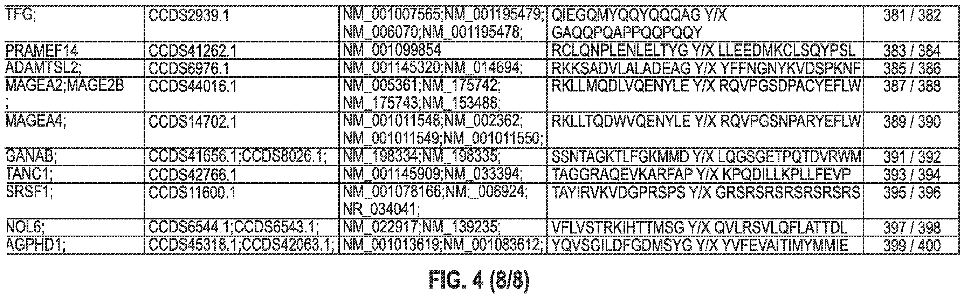

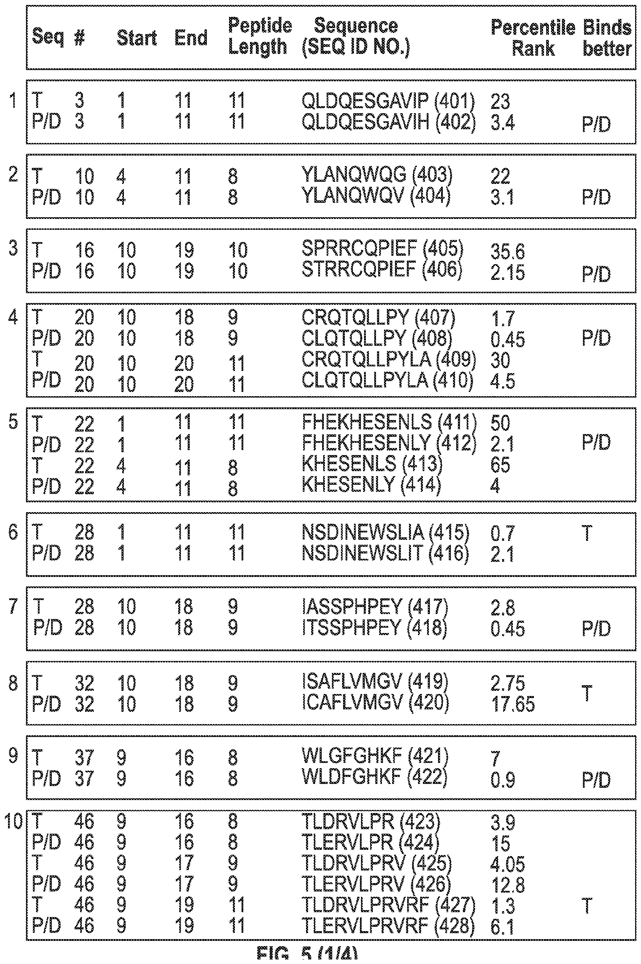

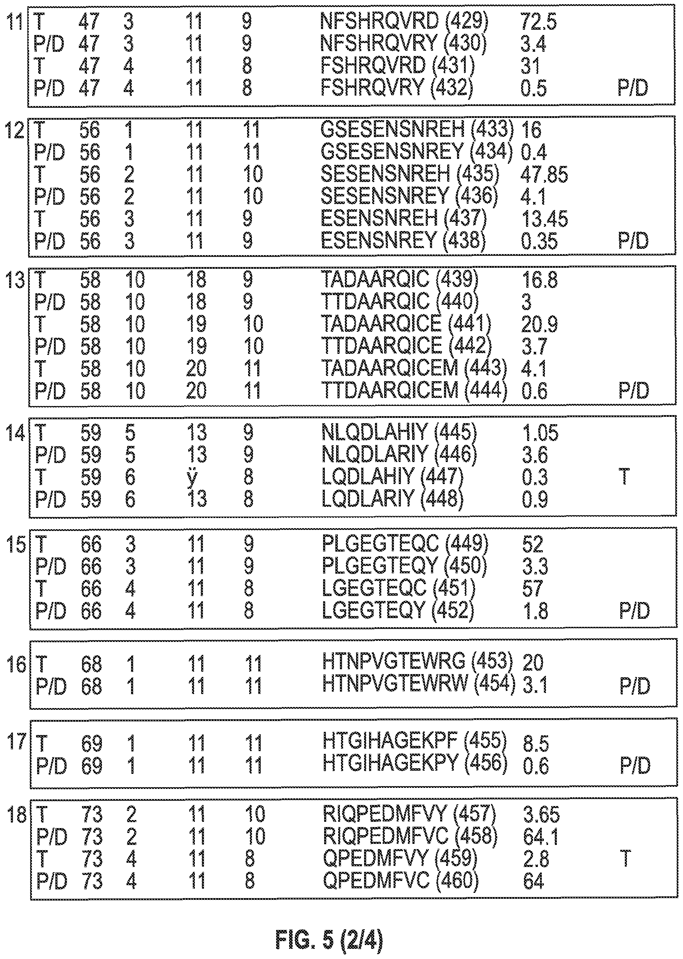

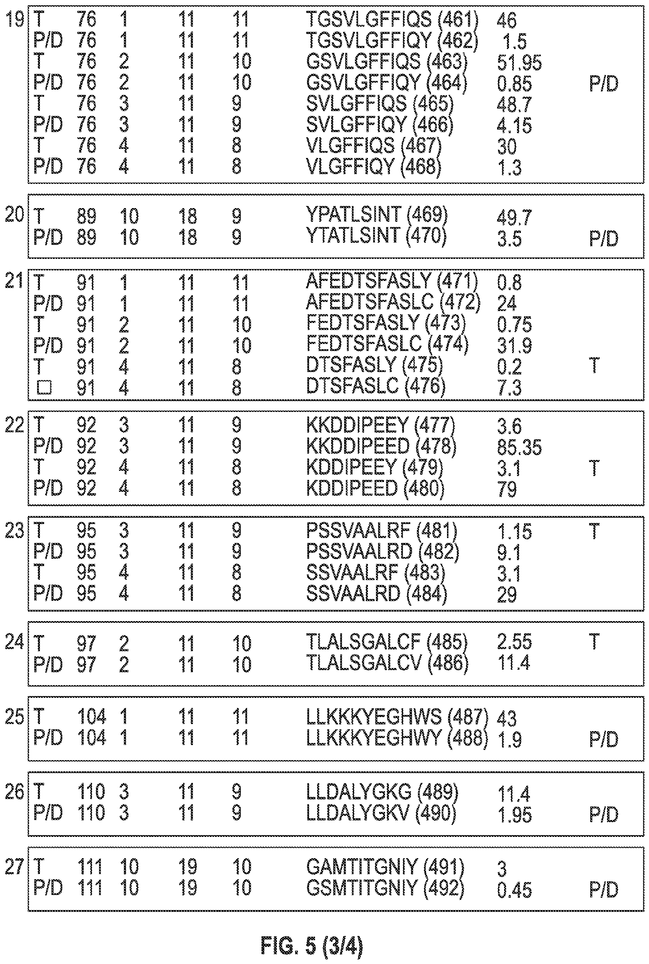

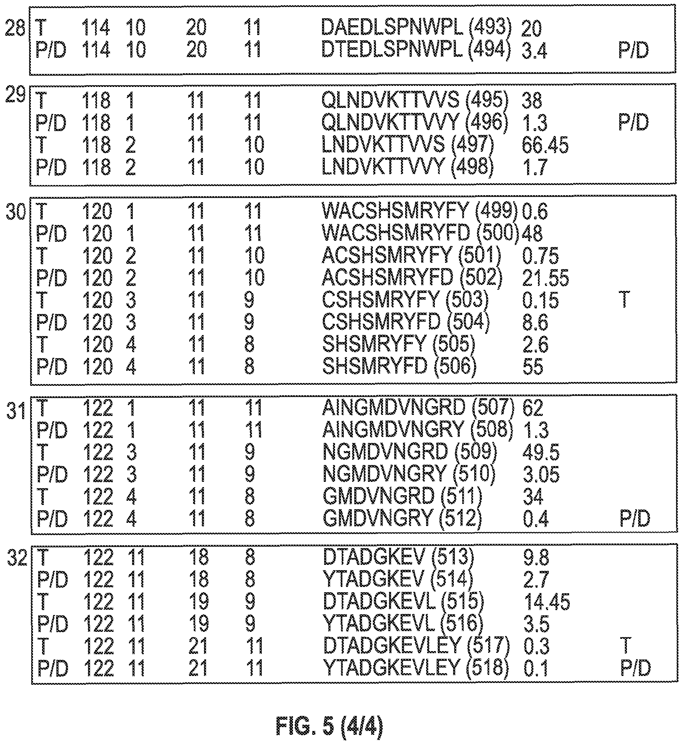

Also provided herein are mutant sequences associated with cancer, wherein the sequence is selected from any disclosed in FIGs. 4 and 5.

Further provided are cancer vaccines prepared using one or more of the cancer antigens identified by any of the above methods. The cancer vaccine may be a polypeptide that contains one or more of the cancer antigens or may be a nucleic acid that encodes for expression of one or more of the cancer antigens.

Yet further provided is a method of treating a cancer patient by identifying cancer antigens from nucleic acid obtained from cancer cells as described by any of the methods above and by preparing a vaccine with one or more of the cancer antigens. The patient is treated by administering the vaccine to generate CTLs in the patient and/or by administering CTL cell lines prepared in vitro by contacting mononuclear cells of the cancer patient or an HLA-matched donor with the cancer antigen vaccine, or by immunizing the donor with the vaccine and transferring immunized donor CTLs to the cancer patient. The contacting may include the addition of autologous CD4+ T cells and/or dendritic cells from the cancer patient or from the HLA-matched donor.

In another embodiment, cancer patients are treated by identifying cancer antigens from nucleic acids obtained from cancer cells as described by any of the methods above, by preparing a vaccine with one or more of the cancer antigens, by contacting mononuclear cells of the cancer patient or an HLA-matched donor (matched for HLA class and/or supertype) with the cancer antigen vaccine to stimulate CTL cell lines, and by administering the CTL cell lines to the cancer patient to treat the cancer . The contacting may include the addition of autologous CD4+ T cells and/or dendritic cells from the cancer patient or from the HLA-matched donor.

### BRIEF DESCRIPTION OF THE DRAWINGS

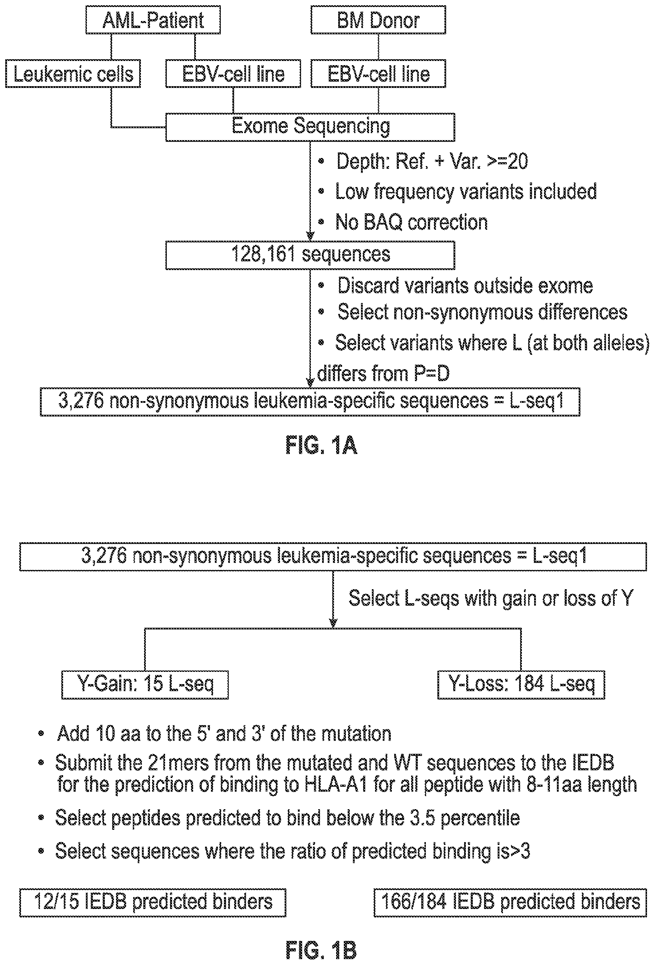

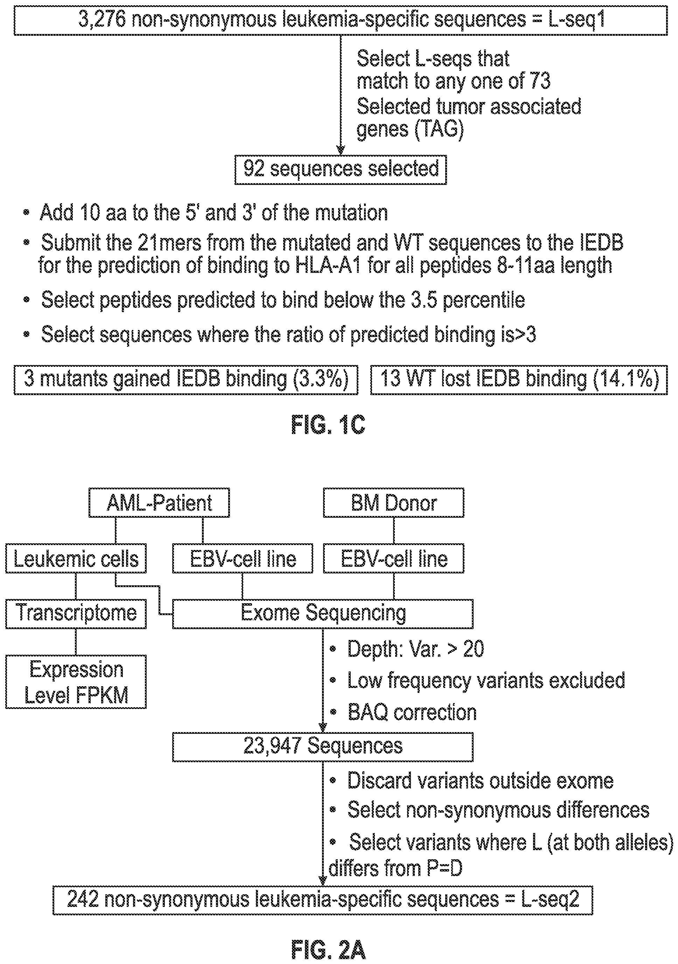

FIG. 1a-c is a set of flow diagrams relating to the identification of mutations in acute myelogenous leukemia (AML) cells from an HLA-A1 patient. FIG. 1a shows the initial selection of mutant sequences determined by applying next generation sequencing to nucleic acid prepared from leukemic cells, EBV-transformed cells from the cancer patient, and EBV-transformed cells from an HLA-matched donor. An initial set of 128,161 sequences were obtained and from that, a set of 3,276 (designated "L-seq1") were selected which have a change of a coded amino acid in a gene from the cancer cell compared to that in EBV cells from the patient and the donor. FIG. 1b shows further selection of the mutant sequences from the L-seq1 set for mutants that involve either a gain or loss of a tyrosine in the protein from the patient cancer cells. Peptide sequences containing the tyrosine involved mutant sequence were tested for binding to HLA-A1 antigens in silico using HLA peptide binding software. FIG. 1c shows selection of the mutant sequences from the L-seq1 set for proteins that have been reported to be associated with cancer. Peptide sequences containing the mutations present in genes that are associated with cancer were tested for binding to HLA-A1 antigens in silico using HLA peptide binding software. The abbreviated terms are as follows: L: leukemia; P: patient; D: donor; ref: reference; var: variant; IEDB: immune epitope database.

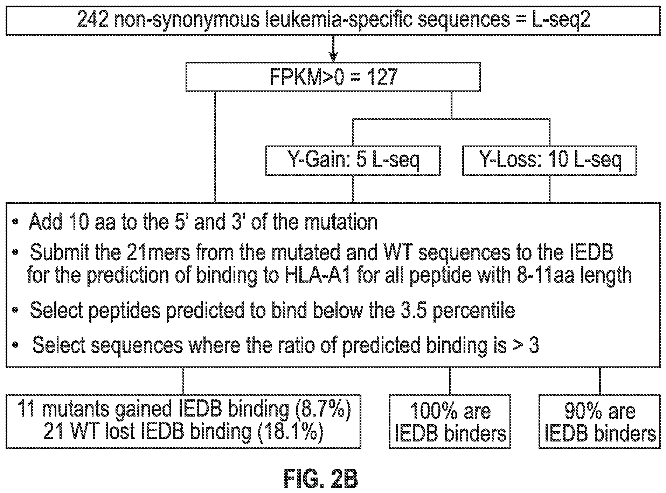

FIG. 2a-b is a second set of flow diagrams relating to the identification of mutations in the same acute myelogenous leukemia (AML) cells and donor cells used in FIG. 1a-c. FIG. 2a shows selection of an initial set of 23,947 sequences and from that, a set 242(designated "L-seq2") were selected which have a change of a coded amino acid in a gene from the leukemia cell compared to that in EBV cells from the patient and the donor. FIG. 2b shows further selection of the mutant sequences from the L-seq2 set for mutants that associated with genes expressed by the leukemia cells (FPKM> 0) or involve either a gain or loss of a tyrosine in the protein from the patient leukemia cells. Peptide sequences containing the tyrosine involved mutant sequence or containing the expressed mutant sequence were tested for binding to HLA-A1 antigens in silico using HLA peptide binding software. The acronyms referred to are as follows: L: leukemia; P: patient; D: donor; ref: reference; var: variant; IEDB: immune epitope database.
FIG. 3 shows the difference in amino acid distributions for the patient and donor (P=D) EBV cells and the leukemia cells for the 3,276 sequences in the L-seq1 set (L differs at both alleles from P=D). The highlighted amino acids are involved in binding to HLA-A1.
FIG. 4 identifies proteins in L-seq1 with amino acid changes involving a tyrosine and provides a 31 amino acid peptide sequences for patient and donor (P/D) and corresponding leukemic cell (L) of the cancer patient.
FIG. 5 identifies peptides from 32 proteins from L-seq2 and provides the sequence in leukemic cancer cells (T) and the corresponding sequence in the patient and donor (P/D). Also provided is an HLA-A1 binding ranking for each sequence.
FIG. 6 identifies 73 tumor associated genes.
FIG. 7 identifies various HLA supertypes and mutant and wildtype position amino acids that can be used for selecting mutant sequences identified by NGS.

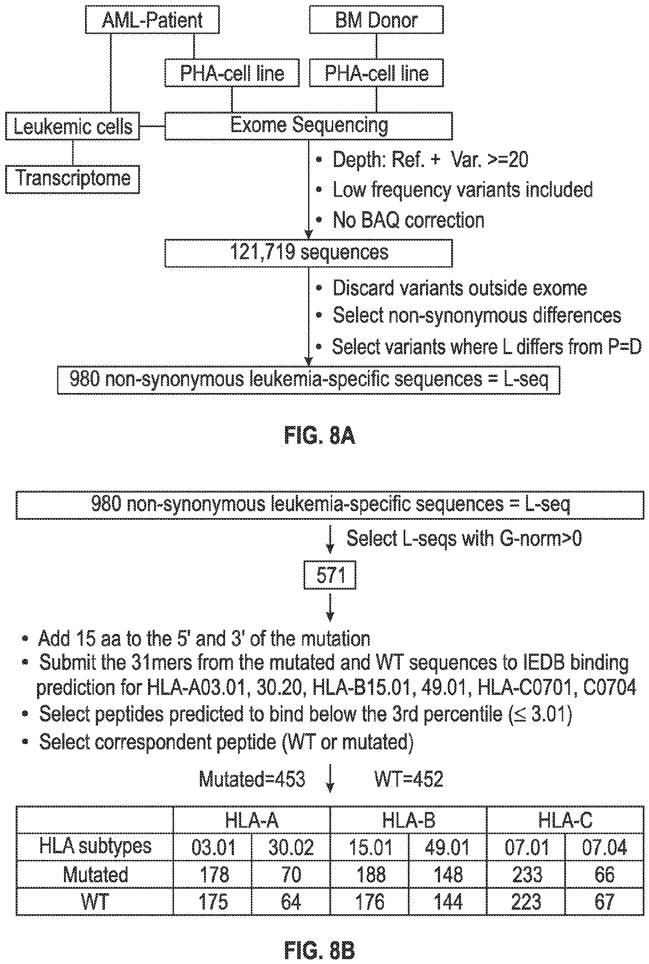

FIG 8 is a set of flow diagrams relating to the identification of mutations in acute myelogenous leukemia (AML) cells from patient #2 as described in Example 3. FIG. 8a shows the initial selection of mutant sequences determined by applying next generation sequencing to nucleic acid prepared from leukemic cells, PHA-stimulated lymphocytes from the cancer patient, and PHA-stimulated lymphocytes from an HLA-matched donor. An initial set of 121,719 sequences were obtained and from that, a set of 980 (designated "L-seq") were selected which have a change of a coded amino acid in a gene from the cancer cell compared to that in PHA-stimulated lymphocytes from the patient and the donor. FIG. 8b shows further selection of the mutant sequences from the L-seq that are expressed as measured by transcriptome analysis. The L-seq set of 980 31-mers were tested for binding to various HLA subtypes in silico using HLA peptide binding software. It was predicted that 571 of those 31-mers would exhibit HLA binding activity to at least one HLA subtype by at least one region of the peptide. There were a total of 905 predicted binding regions, of which 452 were wild-type sequences and 453 were mutated sequences. The Table insert shows the number of peptides predicted to bind to each specific HLA. The total number of HLA-binding sequences in table insert is greater than 571 because several peptides bound to more than one HLA allele.

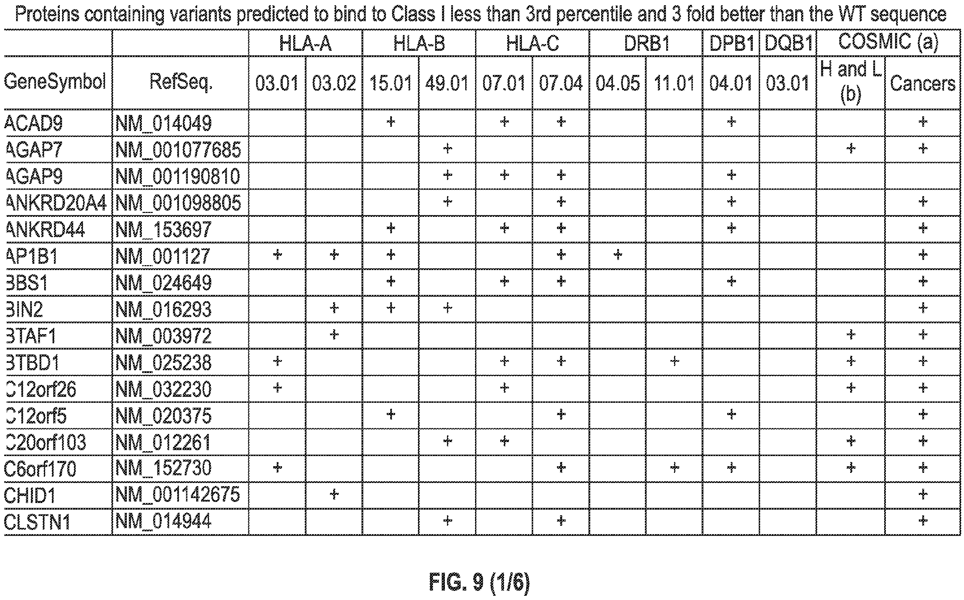

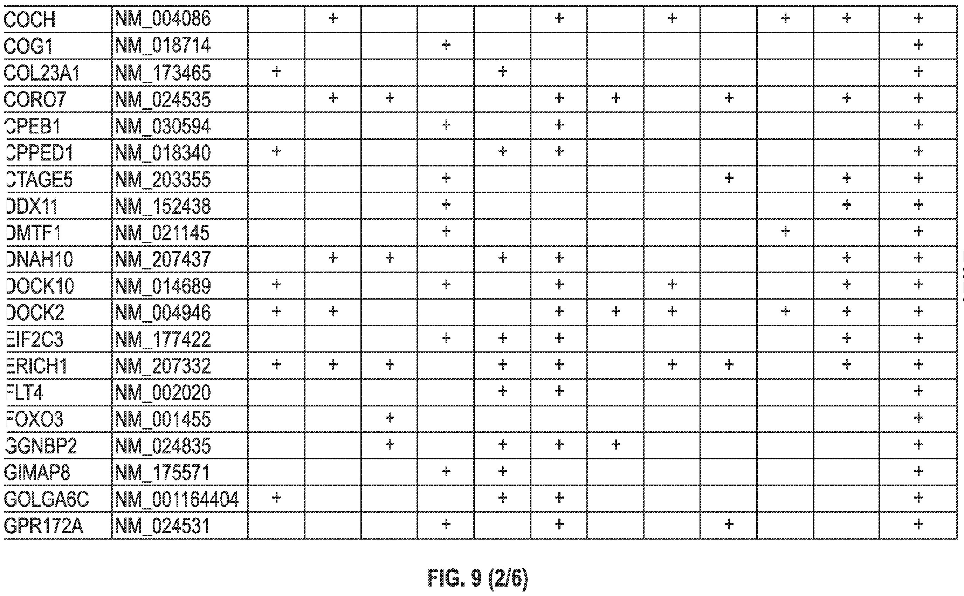

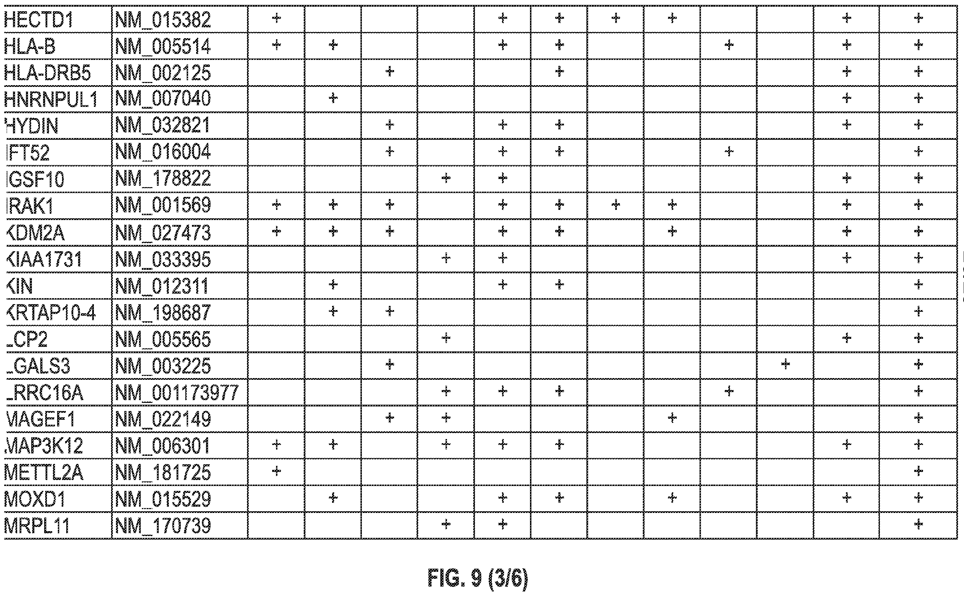

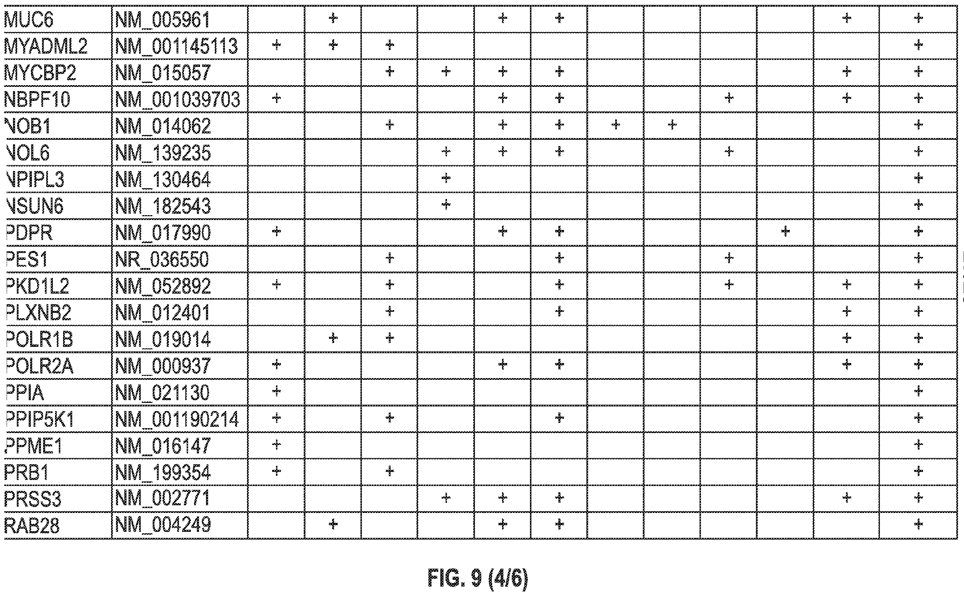

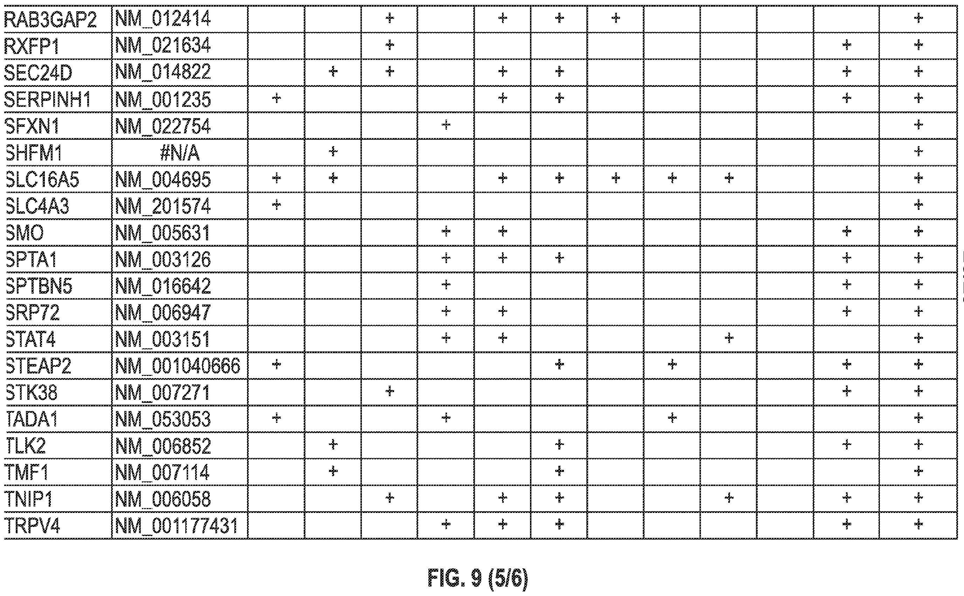

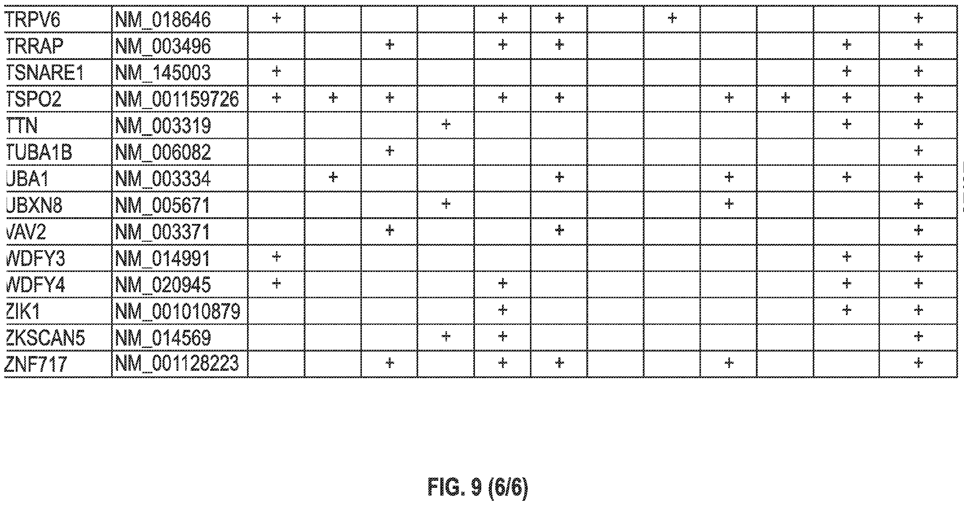

FIG 9 shows the list of mutated proteins where the mutation resulted in a predicted binding affinity of less than 3% and a 3-fold increase of binding as compared to the equivalent wild type peptide. Each line represents the binding analysis of one 31mer containing the mutation. Binding to more than one MHC might indicate that within the 31mer, more than one peptide sequence is responsible for the binding. (a) Catalog Of Somatic Mutations=COSMIC and (b) H and L= hematopoietic and lymphoid tissues.

### DETAILED DESCRIPTION OF THE INVENTION

Identification of T cell antigens that can be used for immunotherapy still faces many problems. The search for the antigens has been very laborious and after an antigen is discovered, there is a strong tendency to generalize an antigen's applicability, assuming that an antigen that works for one individual will be an antigen to treat the same kind of tumor in another individual. This tendency is based on the notion that, to be useful, an antigen needs to work in the broadest possible patient population. This practice of "generalizing" tumor antigens does not account for the fact that each tumor expresses many unique antigens and that an individual's MHC molecules restrict the T cell response. Therefore, an antigen that is good for treating one individual might not be ideal for another person. Moreover, many of the antigens identified so far are normal tissue specific antigens, raising the problem of autoimmunity.

The present paradigm for the discovery and immunotherapeutic application of tumor antigens is to look for "universal" or common tumor antigens, i.e., antigens that induce good immunity in the majority of individuals and use these antigens for vaccination purposes. The results obtained with these approaches have been disappointing. The reality is that the best immune response will differ for each patient affected by a tumor. Only after the immune repertoire is identified for many individuals, using a systematic and unbiased approach, would it be possible to ascertain common patters of immunogenic mutations. For example, a finding that some genes were affected by common clusters of mutations may lead to the application of a less individualized therapy.

Provided herein are methods to identify the available mutations that constitute tumor-associated and/or tumor-rejection antigens in cancer cells from individual cancer patients and to use the antigens to immunize T cells from the patient or HLA-matched donor T cells to recognize and kill the cancer cells. To this end, next generation sequencing will be used to sequence the transcriptome and the exome of the cancer cells as well as the exome of EBV lymphoblasts, or PHA stimulated T cells from the cancer patient. In particular cancers that employ bone marrow transplantation (e.g. in leukemia), the sequenced exome from the cancer patient cells also can be compared to the exome from EBV lymphoblasts or PHA-stimulated cells from an HLA-matched bone marrow donor. This comparison will yield a comprehensive representation of the genes that will be translated into peptides and utilized in adoptive transfer therapy.

Next generation sequencing strategies make it possible to perform extensive molecular characterization of tumor cells in an attempt to identify genes involved in transformation. The information that can be obtained includes the copy number, level of expression, and somatic mutations.

As used herein, the terms "next generation sequencing" ("NGS"), "second generation sequencing" and "massively parallel sequencing" encompasses high-throughput sequencing methods that parallelize the sequencing process, producing thousands to millions of sequences at a time (16, 17). The number of sequences produced by parallelized sequencing is typically greater than 10,000, more typically greater than 100,000 and most typically greater than 1 million. NGS design is different from that of Sanger sequencing, also known as "capillary sequencing" or "first-generation sequencing," which is based on electrophoretic separation of chain-termination products produced in individual sequencing reactions.

Although NGS platforms differ in engineering configurations and sequencing chemistry, common to most is the use of spatially separated, clonally amplified DNA templates or single DNA molecules processed in parallel by use of a flow cell. The massive quantity of output from parallel processing is transformed from primary imaging output or detection output into sequence. A package of integrated algorithms performs the core primary data transformation steps: image analysis, intensity scoring, base calling, and alignment of subsequence reads to a reference sequence. Reference sequences include the human reference genome NCBI37/hg19 sequence, which is available from Genome Bioinformatics Group of the University of California Santa Cruz (available on the world wide web). Other sources of public sequence information include GenBank, dbEST, dbSTS, EMBL (the European Molecular Biology Laboratory), and the DDBJ (the DNA Databank of Japan). Thus, NGS refers to a parallel sequencing platform that employs parallel processing of nucleic acid leading to sequence reads and mapping of the sequence reads to a database with reference gene sequences.

The present methods for selecting cancer specific sequences using NGS have the potential to identify rare mutants which may be lost when more extensive sequence filtering is used. Even a single tumor may contain different types of cancer cells and different types of stem cells that continue to replicate themselves and also give rise to the these types of cancer cells. The exclusion of filtering between the step of sequence alignment and selection of mutant sequence will likely result in more false positives but will provide rare sequence mutations that need to be immunized against to treat the cancer.

Sequence data from NGS systems can be filtered to provide early selective criteria which aids in accuracy. A common filter is the "depth of coverage," "sequencing coverage" or "coverage depth," which is the average number of times a given DNA nucleotide is represented in sequence reads (stated differently, this is the average number of reads covering any particular base) (see, e.g., Nielsen et al., Nature Reviews Genetics 12:443 2011) The greater the coverage, the greater the likelihood of accurately calling a sequence variation. For detection of cancer mutations as disclosed herein, a depth of coverage of < 20 x is used, however, more preferable coverage is < 15 x, < 10 x, < 7 x, < 5 x, < 4 x, < 3 x, < 2 x, and 1 x. Depth of coverage also can be represented in the case of mutations as the average number of reads for the reference and variant combined for any particular base (e.g., the reference is the patient or donor EBV B cell sequences and the variant is the cancer cell sequences. A reference + variant(s) >=20 can be used to identify with confidence low depth for the cancer mutation (e.g. 18 x for the reference plus 2 x for the mutation).

Another filter is base alignment quality (BAQ), an approach that accurately measures the probability that a read base has been wrongly aligned (e.g., see Li, Bioinformatics; 27(8): 1157-1158 [2011]). Base alignment quality (BAQ) computation is turned on by default and adjusts depth of coverage values to better simulate local realignments.. BAQ is a Phred-like score representing the probability that a read base is misaligned; it lowers the base quality score of mismatched reads that are near indels. This is to help rule out false positive SNP calls due to alignment artifacts near small indels. The filter can be adjusted by utilizing its parameters. One can disable BAQ with the -B parameter, or perform a more sensitive BAQ calculation with -E.

CodonCode Corporation (58 Beech Street Dedham, MA 02026) offers Windows, Mac OS X, and Unix versions of Phrap, Phred, and Cross_match, Phil Green's programs for sequence assembly, quality base calling, and fast sequence comparisons. CodonCode also offers Unix and Linux versions of Consed, David Gordon's contig editor and automated finishing tool for Phred and Phrap. After calling bases, Phred examines the peaks around each base call to assign a quality score to each base call. Quality scores range from 4 to about 60, with higher values corresponding to higher quality. The quality scores are logarithmically linked to error probabilities, as shown in the following table:

| **Phred quality score** | **Probability that the base is called wrong** | **Accuracy of the base call** |
|---|---|---|
| 10 | 1 in 10 | 90% |
| 20 | 1 in 100 | 99% |
| 30 | 1 in 1,000 | 99.9% |
| 40 | 1 in 10,000 | 99.99% |
| 50 | 1 in 100,000 | 99.999% |

Another sequence data filter is probabilistic modeling, which employs algorithms to filter low frequency variants.

NGS sequencing technologies include pyrosequencing, sequencing-by-synthesis with reversible dye terminators, sequencing by oligonucleotide probe ligation and real time sequencing. NGS sequencing technologies are available commercially, such as the sequencing-by-hybridization platform from Affymetrix Inc. (Sunnyvale, Calif.) and the sequencing-by-synthesis platforms from 454 Life Sciences (Bradford, Conn.), Helicos Biosciences (Cambridge, Mass.), Illumina/Solexa (Hayward, Calif.), and the sequencing-by-ligation platform from Life Technologies (San Diego, Calif.). Several companies provide NGS sequencing direct to the consumer for $10,000 or less.

The first well known example of NGS sequencing technology is the 454 (Roche) Life sequencing system (e.g. see Margulies, M. et al. Nature 437:376-380 [2005]). In 454 sequencing, the DNA is first sheared into fragments of approximately 300-800 base pairs, and the fragments are blunt-ended. Oligonucleotide adaptors, which serve as primers for amplification and sequencing of the fragments, are ligated to the ends of the fragments. The adapted fragments are attached to DNA capture beads and the beads are individually PCR amplified within droplets of an oil-water emulsion to yield multiple copies of clonally amplified DNA fragments on each bead. The beads are captured in wells where pyrosequencing is performed on each DNA fragment in parallel. Pyrosequencing makes use of pyrophosphate (PPi) which is released upon nucleotide addition, is converted to ATP by ATP sulfurylase in the presence of adenosine 5' phosphosulfate, the ATP then used to convert luciferin to oxyluciferin, generating light that is detected and measured.

Other exemplary NGS sequencing technologies include the Helicos True Single Molecule Sequencing (tSMS) (e.g. see Harris T. D. et al., Science 320:106-109 [2008]); the nanopore sequencing method (e.g. see Soni G V et al., Clin Chem 53: 1996-2001 [2007]); the chemical-sensitive field effect transistor (chemFET) array (e.g., see U.S. Patent Application Publication No. 20090026082); the Halcyon Molecular's method that uses transmission electron microscopy (TEM) (e.g., see PCT patent publication WO 2009/04644); Illumina's sequencing-by-synthesis and reversible terminator-based sequencing chemistry (e.g. see Bentley et al., Nature 6:53-59 [2009]); the SOLiD™ (Life Technologies) sequencing-by-ligation technology (e.g., see McKernan et al., Genome Research 19 (9): 1527-41 (2009]); Pacific Biosciences single molecule, real-time SMRT™ sequencing technology (e.g., see Levene et al., Science. 299 682-686 [2003]); and Life Technologies Ion Torrent single molecule sequencing on a semiconductor chip.

NGS can be used to generate a whole genome sequence or a subset of a whole genome sequence such as an exome sequence or a transcriptome sequence. As used herein, the term "genome sequence" can be referred to as a "genome library" or "genome library of sequences. Likewise, the terms "exome sequence" and "transcriptome sequence" can be referred to as an "exome library" or "exome library of sequences, or "transcriptome library" or "transcriptome library of sequences, respectively.

A whole genomic sequence is obtained by applying NGS to total genomic DNA. The exome represents the protein coding sequences of all genes in the genome. Exome sequence is obtained, for example, by preparing a genomic library and selecting exomic sequence using target-enrichment methods such as hybrid capture or in-solution capture. An exome library may contain intronic or other non-exon sequence as the enrichment may not be total. For example, exome libraries may be only about 50% pure with respect to exon sequences.

The mutant sequences from the cancer cells can be identified by whole transcriptome sequencing using known methods (13-15). The transcriptome is the set of all RNA molecules, including mRNA, rRNA, tRNA, and other non-coding RNA produced in one or a population of cells. The use of next-generation sequencing technology to study the transcriptome at the nucleotide level using cDNA libraries is known as RNA-Seq (e.g., see Wang et al., Nature Rev. Genetics 10(1): 57-63 [2009]). RNA-Seq provides insights at multiple levels into the transcription of the genome as it yields sequence, splicing, and expression-level information leading to the identification of novel transcripts and sequence alterations. Transcriptome sequence is also obtained by target-enrichment methods such as hybrid capture or in solution capture applied to RNA (e.g. oligonucleotide "bait" capture). RNA-seq does not require a reference genome to gain useful transcriptomic information. RNA-Seq approaches (e.g. see SOLiD™ Whole Transcriptome Analysis Kit from Applied Biosystems, Life Technologies Corporation) preserves strand specificity and can interrogate either polyA or ribo-depleted RNA. However, transcriptome sequences can be mapped to the RefSeq's mRNA database of the National Center for Biotechnology Information (NCBI).

The level of expression of gene sequence from NGS can be obtained by evaluation of NGS performed on the transcriptome library. The unit FPKM (expected fragments per kilobase of transcript per million fragments sequenced) provides a numerical value for the estimated proportion of each transcript.

Recent publications report that transcriptome sequencing, analyzing the complementary DNA (RNA-seq) (18), can be performed without the necessity to clone cDNA libraries or even to simply amplify the mRNA (19). The new methods eliminate steps that otherwise may incorporate errors due to RNA/DNA amplification and cloning. These new methods not only allow the detection of mutations but are also becoming an alternative to microarrays in studies involving gene expression and copy number alterations in genome-wide analysis.

Cancers suitable for analysis generally include carcinomas, leukemias or lymphomas, and sarcomas. Carcinomas may be of the breast, colon, rectum, lung, oropharynx, hypopharynx, esophagus, stomach, pancreas, liver, gallbladder and bile ducts, small intestine, urinary tract, female genital tract, male genital tract, endocrine glands, and skin. Other suitable cancers include hemangiomas, melanomas, and tumors of the brain, nerves, eyes, and meninges.

Sequence reads coming from NGS may code for all or a portion of an expressed gene in the cancer cell. Mutations of interest can result from a substitution of a wildtype amino acid but may also result from amino acid changes caused by deletions or insertions of nucleotide sequence in the encoding nucleic acid. As used herein, "mutation" means any change in a DNA sequence (or change in amino acid sequence) away from normal. Thus, when there is a normal allele that is prevalent in the population, a mutation changes this to a rare and abnormal variant. In contrast, a "polymorphism" is a DNA sequence variation that is common in the population. In this case no single allele is regarded as the standard sequence. Instead there are two or more equally acceptable alternatives for a wildtype sequence. As used herein, a cut-off point between a mutation and a polymorphism can be 1 per cent. Thus, a polymorphism arises when the least common allele has frequency of at least 1 per cent in the population. If the frequency is lower than 1%, the allele is regarded as a mutation.

Coding sequences from exome and/or transcriptome libraries from the cancer cells are compared to the exome of EBV lymphoblasts (or PHA blasts) or any other cell from the patient excluding the tumor cell from the cancer patient and in some instances from the exome from EBV lymphoblasts (or PHA blasts) from an HLA-matched bone marrow donor. Preferred mutations are those where the sequence in a coding region of a gene in the cancer is different from the same gene in normal cells or essentially wildtype cells (e.g. EBV transformed cells) from the cancer patient and, if used, from the same gene from an HLA-matched bone marrow donor. Preferably, the sequence from a gene in normal cells or essentially wildtype cells (e.g. EBV transformed cells) and from a gene from an HLA-matched bone marrow donor are the same. This approach is exemplified in FIGs 1(a) and 2(a).

Further selection of the mutations identified in coding regions of genes expressed in the cancer cells is achieved by identifying those sequences which have a particular amino acid in the cancer cell gene sequence and/or a particular amino acid at the corresponding position in the wildtype gene sequence. The amino acid at the corresponding position in the gene from the cancer cell and from the corresponding wildtype sequence can be referred to as "mutant position amino acid" and "wildtype position amino acid, respectively.

The selection of sequences based on particular mutant position amino acids and wildtype position amino acids may depend on the nature of the major histocompatibility complex (MHC) class I or class II supertype of the cancer patient. As used herein, MHC refers to a cell surface molecule encoded by a large gene family in all vertebrates. MHC molecules mediate interactions of leukocytes, also called white blood cells (WBCs), which are immune cells, with other leukocytes or body cells and determines compatibility of donors for organ transplant as well as one's susceptibility to an autoimmune disease via crossreacting immunization. In humans, MHC may also be referred to as human leukocyte antigen (HLA).

The MHC gene family is divided into three subgroups-class I, class II, and class III. Diversity of antigen presentation, mediated by MHC classes I and II, is attained in multiple ways: 1) the MHC's genetic encoding is polygenic; 2) MHC genes are highly polymorphic and have many variants; and 3) several MHC genes are expressed from both inherited alleles.

MHC functions to display peptide fragment (epitope) of protein molecules-either of the host's own phenotype or of other biologic entities-on the cell surface for recognition by T lymphocytes (T cells). MHC class II antigens generally mediate immunization-specific immunity-to an antigen while MHC class I antigens generally mediate destruction of host cells displaying that antigen.

HLA class I molecules can be clustered into groups, designated as supertypes, representing sets of molecules that share largely overlapping peptide binding specificity. Each supertype can be described by a supermotif that reflects the broad main anchor motif recognized by molecules within the corresponding supertype.

In accordance with an embodiment of the invention, the large number of mutant sequences initially identified by NGS analysis are further selected by identifying the MHC class I or class II type of the cancer patient and then choosing one or more mutant position amino acids and/or wildtype position amino acids that are changed in the cancer cells. Thus, mutant sequences are selected by identifying an HLA supertype of the cancer patient and then selecting one or more amino acids for the HLA supertype as the mutant position amino acid and/or wildtype position amino acid using FIG. 7. The amino acids in FIG. 7 constitute known amino-acid binding preferences for the HLA pockets for the specified the HLA supertypes (see, for example, Sydney et al. BMC Immunology 2008, 9:1, Ramensee et al. Immunogenetics (1999) 50:213). Amino acids highlighted by bold and with underlining are preferred binding residues. For example, where the cancer patient expresses HLA-A1 as the MHC class I antigen, the particular mutant position amino acid or wildtype position amino acid is an amino acid selected from the group consisting of tyrosine, aspartic acid, glutamic acid, leucine, serine and threonine, more preferably, leucine, serine, threonine and tyrosine, and even more preferably tyrosine. In one embodiment, the initial set of mutant and corresponding wildtype sequences obtained by NGS can be selected for those where the mutant amino acid position and the corresponding amino acid position in the wildtype gene sequence involve a gain or loss of any tyrosine. This selection step is exemplified in FIGs 1(b) and 2(b). The selection of one or more amino acids for the HLA supertype as the mutant position amino acid and/or wildtype position amino acid can be one, two, three, four, five six, seven, eight, nine or 10 amino acids. Selection based on one or two amino acids may be sufficient to narrow the library to a manageable number of candidate mutant sequences.

In some embodiments, one may ignore the HLA class and/or supertype of the individual and make a further selection of the mutants based on particular mutant position amino acids and/or wildtype position amino acids. In this instance, one or more amino acids are selected from the group consisting of tyrosine, phenylalanine, leucine, isoleucine, methionine, valine and alanine. One may select from this group one, two, three, four, five or six amino acids for the selection of mutants without regard to HLA supertype.

Further selection of mutant sequences from the cancer cells that may be potential T cell epitopes for recognition by cytotoxic T lymphocytes is achieved by evaluating peptides containing the mutation sequences for their ability to bind to MHC antigens that are expressed by the cancer patient. The terms "peptide" polypeptide," and "protein" are used interchangeably to refer to a polymer of amino acid residues. These terms also apply to amino acid polymers where all the amino acids are naturally occurring or where one or more amino acid residues is an artificial chemical analog of a corresponding naturally occurring amino acid. Amino acids can be in the L or D form as long as the binding function of the peptide is maintained.

All peptide sequences are written according to the generally accepted convention whereby the α-N-terminal amino acid residue is on the left and the α-C-terminal amino acid residue is on the right. As used herein, the term "N-terminus" refers to the free alpha-amino group of an amino acid in a peptide, and the term "C-terminus" refers to the free α-carboxylic acid terminus of an amino acid in a peptide. A peptide which is N-terminated with a group refers to a peptide bearing a group on the alpha-amino nitrogen of the N-terminal amino acid residue. An amino acid which is N-terminated with a group refers to an amino acid bearing a group on the α-amino nitrogen.

The selection of mutant sequences from the cancer cells that may be potential T cell epitopes for recognition by cytotoxic T lymphocytes can be carried out in silico using computer-based algorithm(s) for predicting IC₅₀ values for peptides binding to specific MHC molecules. Prediction tools that are readily available on-line e.g., the immune epitope database (IEDB) (24, 25), the NetMHC-3.0 (26), and the SYFPEITHI database (Ramensee Immunogenetics 1999) can be used to predict the peptide sequences. For the IEDB, a percentile rank is generated for each peptide using three methods (ANN, SMM_align and Sturniolo) by comparing the peptide's score against the scores of five million random 15mers selected from the SWISSPROT database. The percentile ranks for the three methods are then used to generate the rank for a consensus method. A small numbered percentile rank indicates a high affinity T cell epitope. A ratio between the probability of being a binder versus a non-binding also is used to evaluate binding epitopes (see, e.g., US Patent Publication 2012/0070493.

T cell epitopes presented by MHC class I molecules are typically peptides between 8 and 11 amino acids in length, whereas MHC class II molecules present longer peptides, 13-17 amino acids in length. Notwithstanding, the only practical limitation on the T cell epitopes is that they are capable of binding to the MHC molecules, which may be determined empirically, if necessary. On-line T cell epitope prediction programs are extremely accurate, with peptide sequences predicted to an accuracy of 95% (26).

The selection of peptides with useful T cell epitopes may be determined by synthesizing the peptides and testing them for binding to antigen-presenting cells that express the MHC antigens (see, for example, Peters et al. June 2006. PLoS Computational Biology 2:6 e65). Prioritization of the peptides used to test specificity of the cancer specific T cell lines can be done according to their involvement with oncogenes and then by their affinity to the patients' MHC.

Predicting peptides that bind to Class II MHC antigens can be more difficult than for Class I MHC antigens. This can be addressed for class II MHC by synthesizing longer peptides (e.g. 31mers) and selecting for binding to class II expressing cells by in vitro experiments. For example, for the ability of the peptide to induce class II restricted T cells or the ability to serve as helper epitopes and help in the induction of Class I restricted T cells. Because of their length, the Class II peptides may be more promiscuous and bind to multiple Class II alleles. Within the 31mer, epitopes that bind to multiple Class I alleles can be found.

Peptides that are predicted or shown to bind to MHC class I or class II antigens expressed by the cells of the cancer patient can be tested to determine if they are recognized by cytotoxic T lymphocytes (CTL) cell lines prepared from the cancer patient or from an HLA-matched donor. This can be determined in standard assays. For example, CTL lines prepared from the patient can be tested for cytotoxicity against patient leukemic blasts (LB), PHA-induced T lymphocyte cell lines as well as skin fibroblasts (FB) using published methods (23). CTL lines from the patient also can be tested to determine if they inhibit the growth of non-leukemic hematopoietic progenitor cells (22, table 1). Polyclonal CTLs preparations are enriched for CD8⁺ but may contain some CD4⁺ cells (22). Activation of CTL lines can be measured by determining the level of interferon gamma (IFNγ) secretion using the ELISpot assay, or an ELISA which are readily adaptable to high throughput screening.

These methods are useful to obtain polyclonal, leukemic-specific T cell lines from the cancer patient and also provide for their enrichment or cloning. With these methods, one determines whether particular mutations selected by NGS can activate CTL cell lines from the patient so as to kill the patient's cancer cells but spare normal cells (e.g. EBV lymphoblast) from the cancer patient or from an MHC matched normal donor. The peptides with mutant sequence that activate CTL lead to the identification of mutant genes in the patient cancer cells that may be essential to maintain the leukemic phenotype (driver mutations) or lead to identification of mutations in any other genes not involved in the cancer phenotype (passenger mutations).

Peptides with mutant sequence from the cancer cells that activate the patient's (or HLA-matched individuals) CTL lines specific for the cancer cells can be evaluated for their MHC restriction by using one or more of the following methods: blocking by MHC-specific antibodies, recognition of paired EBV-transformed cell lines that differ by one allele, and/or recognition of a single allele transfectant of the .221 cell line (EBV-transformed cell line with no Class I MHC) (22). Mutant peptides with broad MHC restriction may have application to activate cancer specific CTL from cancer patients that have different MHC class I and class II antigens.

Mutant peptides that activate CTL lines from the cancer patients or that have been selected by other methods, i.e., is predicted to bind to one of the HLA of the cancer patient can be used to induce leukemia-specific CTL in vitro from mononuclear cells from the patient or from an HLA-matched subject. It is desirable to directly stimulate mononuclear cells in vitro using the mutant peptides to induce T cell lines that recognize and kill tumor cells. This would avoid the need to use cancer cells to stimulate T cell induction (a very desirable feature in the case of solid tumors) and will make the CTL lines available in a more rapid and less expensive manner. Effector cells generated with this method can be tested for recognition of the peptide and the leukemic cell by the ELISpot assay. One can also test the affinity of the CTL lines by determining the number of cells necessary to kill the tumor.

Mutant peptide containing sequence that contains an epitope recognized by cancer specific CTL lines derived from the patient or from an HLA-matched donor or that have been selected by other methods, i.e., is predicted to bind to one of the HLA of the cancer patient, can be used in the preparation of a cancer vaccine. Such vaccine represents an immunogenic composition that can be administered to an individual with cancer in order to elicit CTLs that specifically recognize the mutant sequence expressed by cancer cells and result in cancer cell killing. The vaccine composition thus comprises mutant peptides or mutant polypeptides corresponding to tumor specific neoantigens identified by the methods described herein.

A suitable vaccine will preferably contain at least one mutant peptide sequence, and more preferably multiple mutant peptide sequences such as 2, 3, 4, 5, 6, 7, 8, 9, 10, or more. The mutant peptide antigens that are used in the vaccine are chosen for their ability to bind to MHC antigens expressed by the cancer patient who is to receive the vaccine. Better, they are peptides that have been recognized by CTL lines specific for the tumor or can induce CTL lines that are specific for the tumor.

The vaccine can comprise a mixture of different peptide sequences or a single polypeptide that comprises a number of mutant sequences, the latter also referred to as a polyprotein. The peptides or polyprotein can be prepared by peptide synthesis chemistry. For proteins that exceed about 50 amino acids in length, the cost of efficiency of peptide synthesis may require that the polypeptide or polyprotein be produced by recombinant DNA expression methods well known in the art such as expression systems in bacteria and yeast as described previously (see, e.g., U.S. Pat. No. 5,116,943). In general, nucleic acid encoding the mutant peptide sequence can be cloned into an expression vector for high yield expression of the encoded product. The expression vector can be part of a plasmid, virus, or may be a nucleic acid fragment. The expression vector includes an expression cassette into which the nucleic acid encoding the mutant peptide sequence is cloned in operable association with a promoter. The expression cassette may also include other features such as an origin of replication, and/or chromosome integration elements such as retroviral LTRs, or adeno associated viral (AAV) ITRs. If secretion of the mutant peptide sequence is desired, DNA encoding a signal sequence may be placed upstream of the nucleic acid encoding the mature amino acids.

Cells suitable for replicating and for supporting expression of the mutant peptide sequences are well known in the art. Such cells may be transfected or transduced as appropriate with the particular expression vector and large quantities of vector containing cells can be grown for seeding large scale fermenters to obtain sufficient quantities of the mutant peptide sequences for clinical applications. Such cells may include prokaryotic microorganisms, such as E. coli, or various other eukaryotic cells, such as Chinese hamster ovary cells (CHO), insect cells, or the like. Standard technologies are known in the art to express foreign genes in these systems.

The vaccine can also be administered in the form of a nucleic acid vector that encodes the mutant sequence and can express the sequence upon entry of the vector into appropriate cells. A variety of regulatory sequences well known to those of skill in the art are included in the vector to ensure expression of the mutant sequence in the target cells. An exemplary vector can be from a virus such as vaccinia or adenovirus Upon entry into a suitable host cell, the mutant peptide sequences are expressed from the vector and can elicit a host CTL response.

The vector may encode a polyprotein sequence by a "minigene" approach where the sequence encoding multiple mutant peptide sequences (i.e., multiple CTL epitopes) are contained in a single open reading frame with or without linker sequence between the epitopes. Thus, these epitope-encoding DNA sequences are directly adjoined, creating a continuous polypeptide sequence. Additional vector modifications required for efficient gene expression may include the use of introns. The inclusion of mRNA stabilization sequences can also be considered for increasing minigene expression as well as immunostimulatory sequences (e.g., CpGs). An alternative to construction of a minigene is to have the different mutant epitopes under separate expression control such as under a multi-cistronic system or with entirely separate controls such as with separate promoters and the related expression elements.

In some embodiments, the vector encoding for the various mutant peptide sequences also may encode a second protein included to enhance immunogenicity. Examples of proteins or polypeptides that could beneficially enhance the immune response include cytokines (e.g., IL2, IL12, GM-CSF), cytokine-inducing molecules (e.g. LeIF) or costimulatory molecules and helper T cells (see, for example, Vitiello et al. 1995. Journal of Clinical Investigation 95, 341). Expression of these immune enhancing proteins can be achieved by full regulation, partial regulation (e.g., bicistronic expression vector) and by use of separate vectors.

The vaccine can comprise a carrier to enhance the resulting immune response to the peptide mutant sequence. A "carrier" as used herein is a molecule that increases the molecular weight of an antigen thereby rendering the antigen immunogenic. A carrier may be any suitable protein e.g., keyhole limpet hemocyanin, serum proteins such as transferrin, serum albumin, and the like, scaffolding structures such as polysaccharide or antigen-presenting cells such as dendritic cells.

The vaccine can be administered in conjunction with an adjuvant. As used herein the term "adjuvant" refers to any substance that enhances an immune response to an antigen. Thus, an adjuvant is used to modify or augment the effects of a vaccine by stimulating the immune system to respond to the vaccine more vigorously. Adjuvants can include liposomes, lipopolysaccharide (LPS), molecular cages for antigen, components of bacterial cell walls, and endocytosed nucleic acids such as double-stranded RNA (dsRNA), single-stranded DNA (ssDNA), interleukins (e.g., IL-12) and unmethylated CpG dinucleotide-containing DNA (see, e.g., U.S. Pat. No. 6,406,705). Adjuvants may be mixed with the vaccine or may be covalently or non-covalently linked to the mutant sequence peptides or polypeptides.

The mutant peptide vaccine (polypeptide or polypeptide expression vector) can be administered in a sufficient amount to treat a cancer patient that has cancer cells expressing the mutant peptide sequence. Mutant peptide sequences are chosen that will bind to and be presented by MHC antigens expressed by the cancer patient. The administered vaccine will generate CTL in the patient against the cancer cells, which cells will kill the cancer cells thereby treating the patient. Alternatively, or in addition, the patient can be administered CTL cells lines prepared from the cancer patient or from an HLA-matched donor that can specifically kill the cancer cells in the patient. These CTL cell lines can be prepared by contacting in vitro mononuclear cells from the cancer patient or from the HLA-matched donor with the vaccine or with cancer cells from the patient.

As employed herein, the phrase "an effective amount," refers to a dose sufficient to provide concentrations high enough to impart a beneficial effect on the recipient thereof. The specific therapeutically effective dose level for any particular subject will depend upon a variety of factors including the disorder being treated, the severity of the disorder, the activity of the specific compound, the route of administration, the rate of clearance of the compound, the duration of treatment, the drugs used in combination or coincident with the compound, the age, body weight, sex, diet, and general health of the subject, and like factors well known in the medical arts and sciences. Various general considerations taken into account in determining the "therapeutically effective amount" are known to those of skill in the art and are described, e.g., in Gilman et al., eds., Goodman And Gilman's: The Pharmacological Bases of Therapeutics, 8th ed., Pergamon Press, 1990; and Remington's Pharmaceutical Sciences, 17th ed., Mack Publishing Co., Easton, Pa., 1990. Dosage levels typically fall in the range of about 0.001 up to 100 mg/kg/day; with levels in the range of about 0.05 up to 10 mg/kg/day are generally applicable. A composition can be administered parenterally, such as intravascularly, intravenously, intraarterially, intramuscularly, subcutaneously, orally or the like. The composition may be administered as a bolus, or slowly infused.

A therapeutically effective dose can be estimated initially from cell culture assays by determining an IC₅₀. A dose can then be formulated in animal models to achieve a circulating plasma concentration range that includes the IC₅₀ as determined in cell culture. Such information can be used to more accurately determine useful initial doses in humans. Levels of the active ingredient in plasma may be measured, for example, by HPLC. The exact formulation, route of administration and dosage can be chosen by the individual physician in view of the patient's condition.

Cancer patients are treated by the methods of the invention if the patient is cured from the cancer or if the cancer is in remission. As used herein, remission is the state of absence of disease activity in patients with a chronic illness. Thus, a cancer patient in remission is cured of their cancer or the cancer is under control. Thus, cancer may be in remission when the tumor fails to enlarge or to metastasize. Complete remission is the absence of disease active with no evidence of disease as indicated by diagnostic methods, such as imaging, such as CT and PET, and sometimes by bone marrow biopsy. When a cancer patient is put into remission, this may be followed by relapse, which is the reappearance of the cancer. Cancer patients can also be treated by adoptive transfer during relapse.

The mutant (and optionally wildtype) peptide vaccine (polypeptide or polypeptide expression vector) can be contacted in vitro by T cells from the patient or from an HLA-matched donor to stimulate cancer specific CTL cell lines. The CTL cell lines can be expanded to yield sufficient numbers of cells and then administered to the cancer patient to treat the cancer. The in vitro contacting may further include the addition of autologous CD4⁺ T cells and/or dendritic cells from the cancer patient or from the HLA-matched donor. During or after administration of the CTL, the patient may be administered the vaccine to further stimulate CTL activity against the cancer cells in the patient.

The term "CD4⁺ T cells" refers to lymphocytes that produce the CD4 protein and interact with dendritic cells to induce antigen presentation by or maturation of the dendritic cells. CD4⁺ T cells may be isolated from natural sources such as blood, cell lines grown in culture, and CD4⁺ T cell clones.

The term CD8+ T cell refers to lymphocytes that produce the CD8 protein. CD8⁺ T cells that can kill target cells are known as CD8+ cytotoxic T lymphocytes (CTL). CD8⁺ T cells may be isolated from natural sources such as blood, cell lines grown in culture, and CD8⁺ T cell clones.

The term "selective" or "specific", when used in reference to CD8+ CTL means a CD8⁺ CTL that preferentially recognizes and has cytotoxic activity toward a cancer cell, compared to a normal cell. A selective CTL can distinguish, or can be made to distinguish, a target pathologically aberrant cell from a population of non-target cells, and does not substantially cross-react with non-target cells. A pathologically aberrant cell refers to a cell that is altered from the normal due to changes in physiology or phenotype associated with a disease or abnormal condition. A cancer cell is an example of a pathologically aberrant cell.

The term "ex vivo" when used in reference to a cell is intended to mean a cell outside of the body. Therefore, an ex vivo cell culture method involves harvesting cells from an individual. Ex vivo culture methods are applicable to a cell harvested from any tissue or organ of an individual.

The term "in situ" when used in reference to selective CTL activity is intended to mean that selective CTLs can destroy a target pathologically aberrant cell in an intact structure of the body. For example, a selective CTL can destroy a target cell in a heterogeneous population of cells. Specifically, a selective CTL can eliminate a pathologically aberrant cell, such as a tumor cell, from a tissue, such as blood or bone marrow.

The term "sufficient time" when used in the context of inducing the generation of boosting the activity of CD8⁺ CTL refers to the time for processing and presenting of an antigen by dendritic cells, recognition by CD8⁺ CTL of an antigen, and activation of cytotoxic activity. A sufficient time that allows for the completion of this process can vary due to differences in the various cell populations of the methods will result in differences in rates of antigen uptake. Factors that can affect the sufficient time for CD8⁺ CTL induction can include the types of cells in a culture, the purity of various cell types, concentrations of cell types, and whether dendritic cells are immature or are presenting antigen at the time of culture.

As used herein, term "isolated" in reference to a cell refers to when the cell is separated from one or more components with which it is associated in nature. An isolated cell also includes a cell purified from non-cellular tissue components, such as connective tissue fibers. An isolated cell can be, for example, a primary cell, either freshly purified from non-cellular tissue components, or cultured for one or more generation. An example of an isolated cell is a cell that has been separated from blood, such as a cell of a preparation of peripheral blood mononuclear cells (PBMCs).

The term "substantially" unless indicated otherwise means greater than 90%, more preferably greater than 95% and more preferably greater than 99%.

As used herein, term "antigen" means a molecule that can be processed and presented by an antigen-presenting cell and subsequently recognized by a T cell receptor. Such a molecule can be for example, a polypeptide or a peptide.

The term "target", when used in reference to the immune reactivity of a CD8⁺ T cell is any predetermined antigen. A predetermined antigen can be, for example, a cell or polypeptide.

As used herein, the term "naive" when used in reference to a CD8⁺ T cell is intended to mean that a CD8⁺ T cell, has either not been exposed to a particular target cell or antigen in vivo. Therefore, a naive CD8⁺ T cell is exposed to a particular target cell or antigen ex vivo in order for it to be capable of CTL activity selective for the particular target cell or antigen.

As used herein the term mononuclear cell refers to a cell with a single nucleus. Mononuclear cells may be immune cells and may be obtained from any of various sites within the body such as blood, lymph, spleen, lymphnode, thymus and bone marrow.

As used herein, the term "treating" is intended to mean reduction in severity or prevention of a pathological condition mediated by a pathologically aberrant cell. Reduction in severity includes, for example, an arrest or decrease in clinical symptoms, physiological indicators, biochemical markers or metabolic indicators. Prevention of disease includes, for example, precluding the occurrence of the disease or restoring a diseased individual to their state of health prior to disease. Treatment of cancer can reflect a maintenance or reduction in tumor size, the absence of metastases or absence of additional metastases, increased disease free interval or extended survival.

As used herein, the term "effective amount" is intended to mean an amount of CD8⁺ CTL required to effect a decrease in the extent, amount or rate of spread of a pathological condition when administered to an individual. The dosage of a CTL preparation required to be therapeutically effective will depend, for example, on the pathological condition to be treated and the level of abundance and density of the target antigens as well as the weight and condition of the individual, and previous or concurrent therapies. The appropriate amount considered as an effective dose for a particular application of selective CTLs provided by the method can be determined by those skilled in the art, using the guidance provided herein. One skilled in the art will recognize that the condition of the patient needs to be monitored throughout the course of therapy and that the amount of the composition that is administered can be adjusted according to the individual's response to therapy.

The following examples serve to illustrate the present invention. These examples are in no way intended to limit the scope of the invention.

### EXAMPLES

### Example 1: Identifying cancer mutations from Exomic and transcriptomic libraries using NGS.

A scheme for identifying mutations in cancer cells that can be the target for immune recognition is shown in FIG. 1a-c. Leukemic cells (L) and Epstein Barr-transformed B cells (patient EBV-Cell line) (P) were obtained from patient #1 prior to hematopoietic stem cell transplantation (HSCT). Previously published methods were used to isolate from blood the leukemic cells (22, 27) and to prepare EBV cells (see, for example, Caputo JL, et al., J. Tissue Culture Methods 13: 39-44, 1991). An EBV-Cell line also was similarly produced from the bone marrow donor (D). The cells were frozen and maintained in liquid nitrogen.

DNA exome libraries were prepared from the patient #1 leukemic cells, the patient #1 EBV-cell line and from the donor EBV-cell line using NGS methods conducted under contract with Expression Analysis, Inc. (Durham, North Carolina). An RNA transcriptome library was prepared by Expression Analysis, Inc., using the patient leukemic cells. In addition to sequencing, the leukemic cell sample was evaluated for the expression level of known genes using the transcriptome library.

The sequencing results from each library were initially subjected to filtering using depth of coverage for reference + variant >= 20 x. No base alignment quality (BAQ) correction and no probabilistic modeling was used to filter out low frequency variants. Under this approach, the sequences from each cell source which differ from the reference gene sequences (human reference genome NCBI37/hg19 sequence (Genome Bioinformatics Group of the University of California Santa Cruz, available on the world wide web) resulted in an initial set of 128,161 mutations ("the 128K set") (FIG. 1).

The 128K mutation set was further selected using the criteria set below:
1. Discard all variants that arise outside of an exon;
2. Select sequences where the base difference results in a non-synonymous amino-acid (aa) change; and
3. Select sequences where the amino acid at a particular position in a gene of the leukemic cell is different for both alleles versus that in the EBV-cell line from the patient and the donor, and where the amino acid is the same (homozygous or heterozygous) in the EBV-cell line of the patient and the donor. This can be summarized as the amino acid (aa) for L (at both alleles) is different from P and D (L differs at both alleles from P=D).

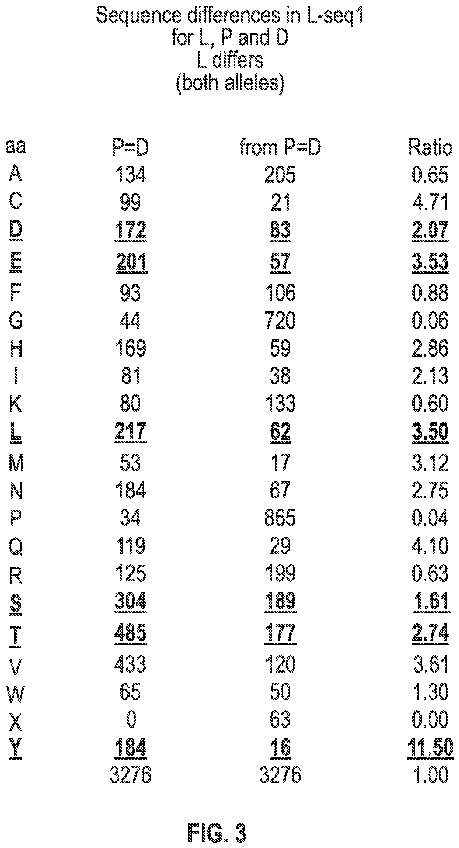

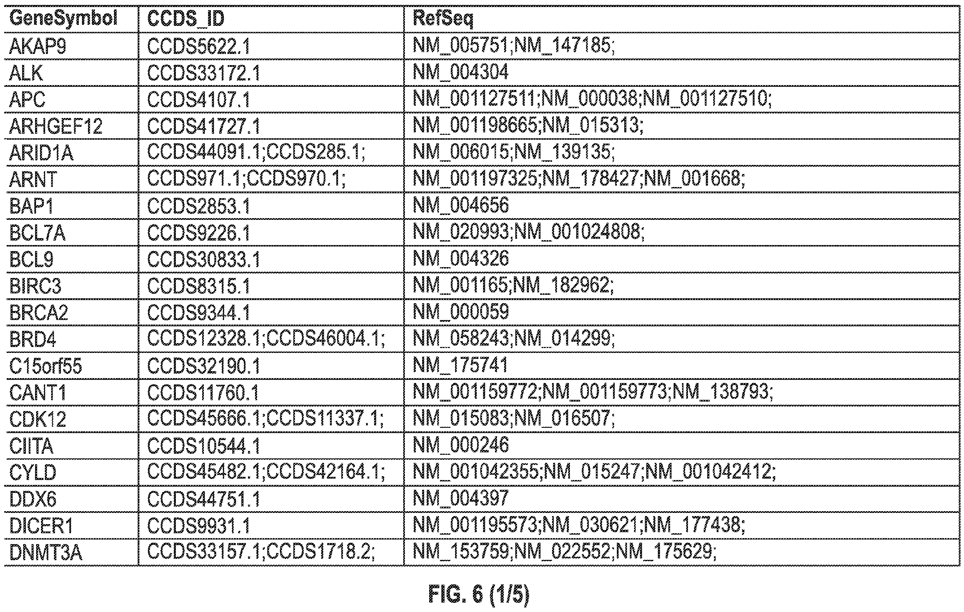

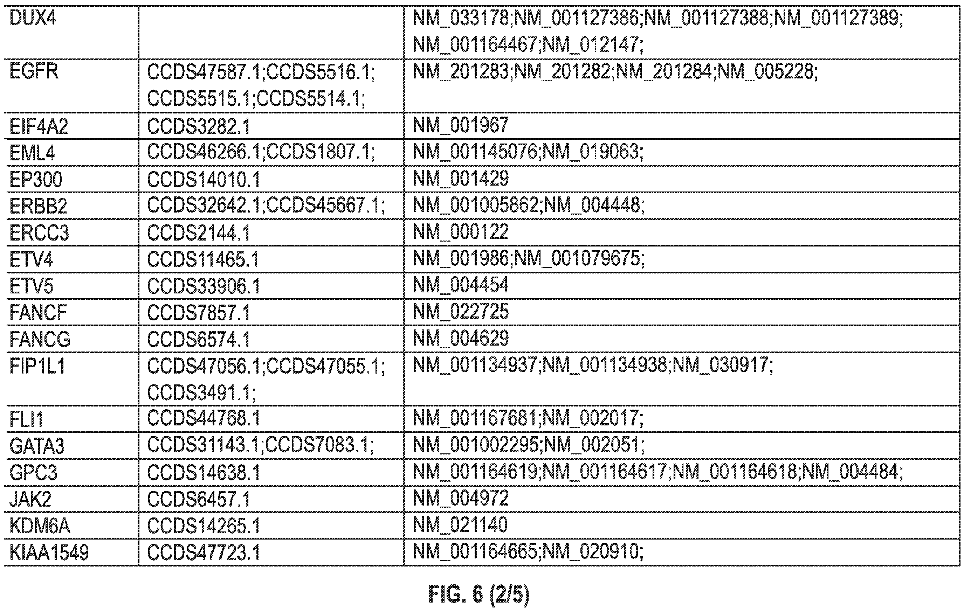

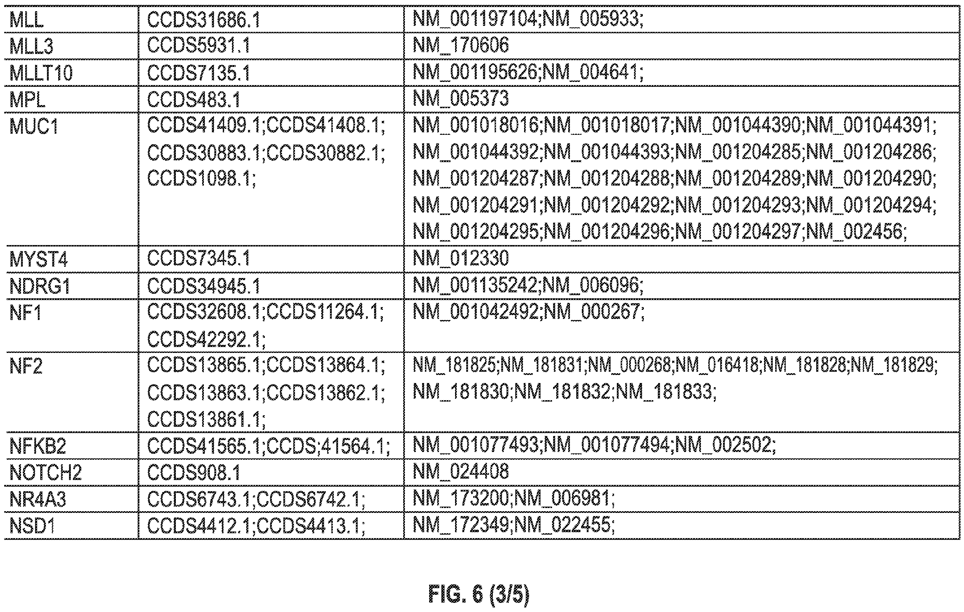

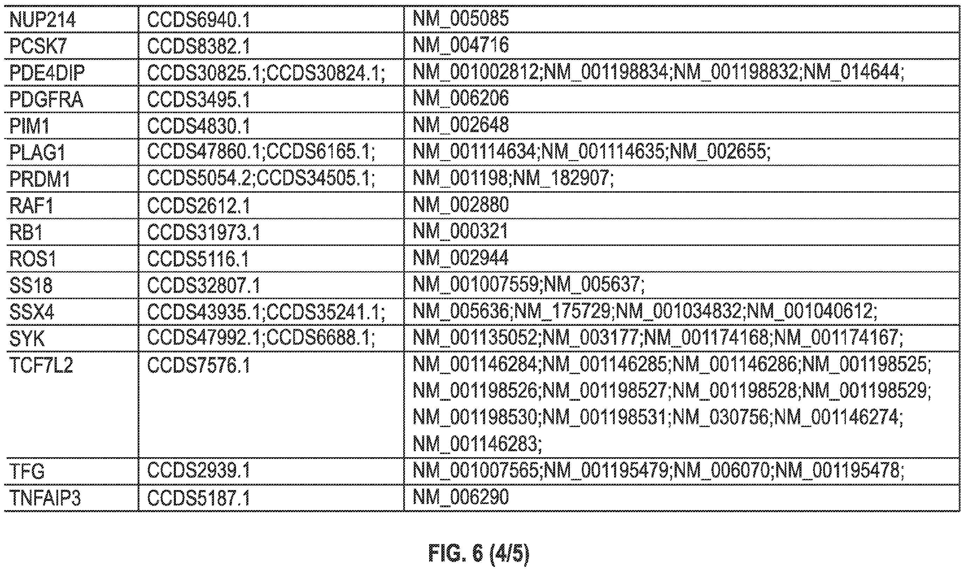

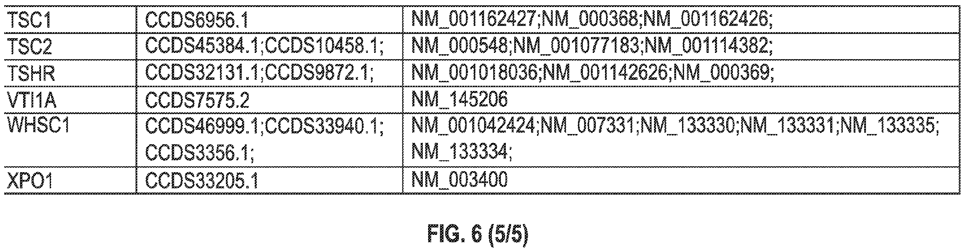

The result of these additional selections applied to the 128K set yielded a smaller set of 3,276 non-synonymous leukemic specific sequences (L-seq1). A further selection of the 3,276 set was obtained by selecting those sequences that are associated with any of a library of 73 known tumor associated genes (TAG) (see FIG. 6). This set of mutations connected with a TAG was then reduced to 92 (FIG. 3).

FIG. 2a-b shows a second approach used to process the raw sequencing information obtained from patient #1. The initial sequences were filtered to a depth of at least 20 reads (>= 20 x), infrequent variants were excluded and BAQ correction was applied. Under this approach, the sequences from each cell source which differ from the reference gene sequences yielded a set of 23,947 (24K) mutants.

The 24K mutation set was further selected using the criteria set below:
1. Discard all variants that arise outside of an exon;
2. Select sequences where the base difference results in a non-synonymous amino-acid (aa) change;
3. Select sequences where the amino acid at a particular position in a gene of the leukemic cell is different for both alleles versus that in the EBV-cell line from the patient and the donor, and where the amino acid is the same (homozygous or heterozygous) in the EBV-cell line of the patient and the donor. This can be summarized as the amino acid (aa) for L (at both alleles) is different from P and D (L differs at both alleles from P=D).

The result of these additional selections applied to the 24K set yielded a smaller set of 242 non-synonymous leukemic specific sequences (L-seq2). This set was further reduced to 127 sequences by selecting only those where the level of expression (FPKM) determined from a transcriptome library was above zero (FIG. 2b).

### Example 2: Selecting cancer specific mutations with potential HLA binding motifs.

The set of mutations from L-seq1 and Lseq2 were further selected to identify a smaller subset with prospects for binding to HLA antigens of the cancer patient. To this end, each L-seq was evaluated for mutants that involve either a gain or loss of a tyrosine. For L-seq1, there were 15 sequences with a tyrosine gain and 184 sequences with a tyrosine loss (FIG. 1b). From the 127 sequences from L-seq2 which were from genes expressed by the cancer cells, there were 5 sequences with a gain of tyrosine and 10 with a loss of tyrosine (FIG. 2b).

Peptide sequences containing the tyrosine involved mutant sequences (both gain and loss) and a corresponding wildtype peptide were transcribed (in silico) as 21mer peptides with 10 amino acids located on each side of the tyrosine involved position. The 21mer peptides were then evaluated for having an 8-11 aa epitope that would exhibit binding to HLA-A1 under the T cell epitope prediction program of IEDB. Peptide sequences were identified that bound below the 3.5% percentile and that showed a ratio of predicted binding greater than 3.

From L-seq1, the gain of tyrosine group showed 12/16 IEDB predicted binders and the loss of tyrosine group showed 166/184 IEDB predicted binders (FIG. 1b). From L-seq2, the gain of tyrosine group showed 5/5 IEDB predicted binders and the loss of tyrosine group showed 9/10 IEDB predicted binders (FIG. 2b). Thus, there is a greater percentage of predicted HLA-A1 binders coming from the tyrosine selected group than from the unselected group screened for tyrosine involved changes. However, the reduced upfront filtering that resulted in the 128K set (versus the 24K set) of prospective mutations resulted in a greater number of mutations which are prospective HLA-A1 binders.

The set of L-seq2 containing 127 expressed cancer specific mutations but not involving a tyrosine change selection showed a lower number of HLA-A1 binders, with 11 acquiring HLA-A1 binding and 21 losing HLA-A1 binding (FIG. 2b). Finally, for the 92 sequences of L-seq1 which were associated with a tumor associated gene, 3/92 acquired HLA-A1 binding while 13/92 lost HLA-A1 binding (FIG. 1c).

FIG. 3 is a listing of the amino acids in patient and donor and corresponding cancer cell of the cancer patient for 3,276 sequences in the L-seq1 set. The highlighted amino acids are those known to be involved in T cell epitopes binding to HLA-A1. The highest P=D to tumor ratio for amino acid changes (gain or loss) was obtained for tyrosine. The other highlighted amino acids also showed significant P=D tumor amino acid change ratios.

FIG. 4 identifies proteins in L-seq1 containing mutant peptide sequence involving a tyrosine and providing a 31mer peptide with mutation involved amino acid located at position 16.

FIG. 5 identifies peptides from 32 proteins from L-seq2 and provides the sequence in cancer cells (T) and the corresponding sequence in the ref (P/D). Also provided is an HLA-A1 binding ranking for each sequence.

### Example 3: Identifying Cancer Mutations - Patient #2

A modified scheme for identifying mutations in cancer cells that can be the target for immune recognition was used on samples obtained from patient #2 and is shown in FIG. 8a. Leukemic cells (L) and Epstein Barr-transformed B cells (patient PHA-Cell line) (P) were obtained from a patient prior to hematopoietic stem cell transplantation (HSCT). Previously published methods were used to isolate from blood the leukemic cells (22, 27) and to prepare EBV cells (see, for example, Caputo JL, et al., J. Tissue Culture Methods 13: 39-44, 1991). An EBV-Cell line also was similarly produced from the bone marrow donor (D). The cells were frozen and maintained in liquid nitrogen.

DNA exome libraries were prepared from the patient leukemic cells, the patient EBV-cell line and from the donor EBV-cell line using NGS methods conducted under contract with Expression Analysis, Inc. (Durham, North Carolina). An RNA transcriptome library was prepared by Expression Analysis, Inc., using the patient leukemic cells. In addition to sequencing, the leukemic cell sample was evaluated for the expression level of known genes using the transcriptome library.

The sequences obtained by the exome sequencing were aligned using BWA (version 0.5.9). with the default parameters except for the seed length (-1) to be 12 bp to aggregate as many alignments as possible. Pileups to use in further downstream processing were generated with Samtools mpilup (http://samtools.sourceforge.net/samtools.shtm) using default parameters, variants were then called using a procedure described previously (Holbrok at al, 2011). The sequences obtained by the transcriptome sequencing were aligned using Bowtie2 and the transcript annotated using the UCSC hg19 reference genome. The RNA transcripts were quantified using RSEM (Ref.: Li, B and Dewey, CN . RSEM: accurate transcript quantification from RNA-Seq data with or without a reference genome. 2011, BMC Bioinformatics 12:323). An initial set of 121,719 mutations ("the 121K set") was obtained.

As described for the patient #1 data, the 121K mutation set from patient #2 was further selected by (1) discarding all variants that arise outside of an exon, (2) select sequences where the base difference results in a non-synonymous amino acid change, and (3) select sequences where the amino acid at a particular position in a gene of the leukemic cell is different for both alleles versus that in the EBV-cell line from the patient and the donor, and where the amino acid is the same (homozygous or heterozygous) in the EBV-cell line of the patient and the donor which can be summarized as the amino acid (aa) for L (at both alleles) is different from P and D (L differs at both alleles from P=D).

The result of these additional selections applied to the 121K set yielded a smaller set of 980 non-synonymous leukemic specific sequences ("L-seq"; FIG. 8a). A further selection of the mutant sequences from the L-seq that are expressed as measured by transcriptome analysis. The L-seq set of 980 31-mers were tested for binding to various HLA subtypes in silico using HLA peptide binding software. It was predicted that 571 of those 31-mers would exhibit HLA binding activity to at least one HLA subtype by at least one region of the peptide. There were a total of 905 predicted binding regions, of which 452 were wild-type sequences and 453 were mutated sequences. The Table insert shows the number of peptides predicted to bind to each specific HLA. The total number of HLA-binding sequences in table insert is greater than 571 because several peptides bound to more than one HLA allele.

### Partial Listing of Cited References

1. 2010. Cancer Facts and Figures 2010. American Cancer Society
2. Dunn, G.P., et al. 2002. Cancer immunoediting: from immunosurveillance to tumor escape. Nat Immunol 3:991-998.
3. Greenman, C., et al. 2007. Patterns of somatic mutation in human cancer genomes. Nature 446:153-158.
4. Kaye, F.J. 2009. Mutation-associated fusion cancer genes in solid tumors. Mol Cancer Ther 8:1399-1408.
6. Muul, L.M., et al. 1987. Identification of specific cytolytic immune responses against autologous tumor in humans bearing malignant melanoma. J Immunol 138:989-995.
7. Rosenberg, S.A., et al. 2008. Adoptive cell transfer: a clinical path to effective cancer immunotherapy. Nat Rev Cancer 8:299-308.
8. Montagna, D., et al. 2006. Emergence of antitumor cytolytic T cells is associated with maintenance of hematologic remission in children with acute myeloid leukemia. Blood 108:3843-3850.
9. van der Bruggen, P., et al. 1991. A gene encoding an antigen recognized by cytolytic T lymphocytes on a human melanoma. Science 254:1643-1647.
10. Boon, T., et al. 1996. Human tumor antigens recognized by T lymphocytes. J Exp Med 183:725-729.
11. Riddell, S.R., et al. 1995. Principles for adoptive T cell therapy of human viral diseases. Annu Rev Immunol 13:545-586.
12. Boon, T., et al. 1994. Tumor antigens recognized by T lymphocytes. Annu Rev Immunol 12:337-365.
13. Haas, B.J., et al. Advancing RNA-Seq analysis. Nat Biotechnol 28:421-423.
14. Lao, K.Q., et al. 2009. mRNA-sequencing whole transcriptome analysis of a single cell on the SOLiD system. J Biomol Tech 20:266-271.
15. Mane, S.P., et al. 2009. Transcriptome sequencing of the Microarray Quality Control (MAQC) RNA reference samples using next generation sequencing. BMC Genomics 10:264.
16. Costa, V., et al. 2010. Uncovering the complexity of transcriptomes with RNA-Seq. J Biomed Biotechnol. 853916.
17. van der Brug, M.P., et al.. Navigating genomic maps of cancer cells. Nat Biotechnol 28:241-242.
18. Nagalakshmi, U., et al. 2008. The transcriptional landscape of the yeast genome defined by RNA sequencing. Science 320:1344-1349.
19. Mamanova, L., et al. FRT-seq: amplification-free, strand-specific transcriptome sequencing. Nat Methods 7:130-132.
22. Montagna, D., et al. 2001. Ex vivo priming for long-term maintenance of antileukemia human cytotoxic T cells suggests a general procedure for adoptive immunotherapy. Blood 98:3359-3366.
23. Montagna, D., et al. 2006. Single-cell cloning of human, donor-derived antileukemia T-cell lines for in vitro separation of graft-versus-leukemia effect from graft-versus-host reaction. Cancer Res 66:7310-7316.
24. Sette, A. 2004. The immune epitope database and analysis resource: from vision to blueprint. Genome Inform 15:299.
25. Zhang, Q., et al. 2008. Immune epitope database analysis resource (IEDB-AR). Nucleic Acids Res 36:W513-518.
26. Lundegaard, C et al. 2008. NetMHC-3.0: accurate web accessible predictions of human, mouse and monkey MHC class I affinities for peptides of length 8-11. Nucleic Acids Res 36:W509-512.
27. Montagna, D., et al. 2003. Generation and ex vivo expansion of cytotoxic T lymphocytes directed toward different types of leukemia or myelodysplastic cells using both HLA-matched and partially matched donors. Exp Hematol 31:1031-1038.

All patents and publications mentioned in the specification are indicative of the levels of those of ordinary skill in the art to which the invention pertains.

## Claims

1. A method of manufacturing a cancer specific cytotoxic T lymphocytes (CTL) cell line for use in treatment of a cancer patient, wherein said method comprises
- identifying cancer antigens from nucleic acid obtained from cancer cells of the cancer patient using the steps of
a) obtaining a plurality of mutant sequences from the nucleic acid of cancer cells from a cancer patient, said mutant sequences coding for all or a portion of an expressed gene and wherein the mutant sequences each have a mutant position amino acid which substitutes for a wildtype position amino acid located at the same position in the wildtype sequence of the protein, wherein said mutant sequences are obtained using a parallel sequencing platform, said parallel sequencing platform employing parallel processing of said nucleic acid of cancer cells leading to sequence reads and mapping of the sequence reads to a database with reference gene sequences; and
b) selecting mutant sequences from those identified in step a) by identifying an HLA class or supertype of the cancer patient and then selecting an amino acid for said HLA class or supertype as the mutant position amino acid using FIG. 7;
wherein cancer antigens are identified in that peptides are synthesized comprising said mutant sequences from step b) and evaluated for activating cytotoxic T lymphocytes (CTL) cell lines prepared from the cancer patient or from an HLA-matched donor, said CTL cell lines obtained by contacting mononuclear cells from the cancer patient or from the HLA-matched donor with cancer cells from the cancer patient;
- contacting T cells from the cancer patient or from a HLA matched donor with the cancer antigens in vitro to stimulate cancer specific cytotoxic T lymphocytes (CTL) cell line.

2. The method of claim 1, wherein said contacting further includes the addition of autologous CD4⁺ T cells and/or dendritic cells from the cancer patient or autologous CD4⁺ T cells and/or dendritic cells from the HLA-matched donor.

3. The method of claim 1, wherein the ability to bind to HLA histocompatibility antigens is carried out in silico using computer-based algorithm(s) for predicting HLA binding peptides.

4. The method of claim 1, wherein peptides comprising the selected sequences from step b) are evaluated for their ability to bind to HLA histocompatibility antigens.

5. The method of claim 4, wherein the ability to bind to HLA histocompatibility antigens is carried out in silico using computer-based algorithm(s) for predicting HLA binding peptides.

6. The method of claim 4, wherein the ability to bind to HLA histocompatibility antigens is carried out by synthesizing the peptides and testing them for binding to antigen-presenting cells that express HLA histocompatibility antigens.

7. The method of claim 1, wherein said parallel sequencing platform filters the sequencing results using a depth of coverage less than 20 x and/or by not filtering with a base alignment quality (BAQ) algorithm.

8. The method of claim 1, wherein the HLA class or supertype is HLA-1 and the mutant amino acid or wildtype amino acid is selected from the group consisting of tyrosine, aspartic acid, glutamic acid, leucine, serine and threonine, and wherein the cancer patient expresses the HLA-A1 histocompatibility antigen.

9. The method of claim 1, wherein said mononuclear cells are enriched in CD8⁺ cells.

10. The method of claim 1, wherein said contacting further includes the addition of autologous CD4⁺ T cells and/or dendritic cells from the cancer patient or autologous CD4⁺ T cells and/or dendritic cells from the HLA-matched donor.

## Patentansprüche

1. Verfahren zur Herstellung einer Zelllinie von krebsspezifischen zytotoxischen T-Lymphozyten (CTL-Zelllinie) zur Verwendung bei der Behandlung eines Krebspatienten, wobei das Verfahren umfasst:
- Identifizieren von Krebsantigenen aus von Krebszellen des Krebspatienten erhaltener Nukleinsäure unter Verwendung der folgenden Schritte:
a) Erhalten einer Mehrzahl von mutierten Sequenzen aus der Nukleinsäure von Krebszellen von einem Krebspatienten, wobei die mutierten Sequenzen alles oder einen Teil von einem exprimierten Gen codieren und wobei die mutierten Sequenzen jeweils eine Aminosäure an der mutierten Position aufweisen, die eine Aminosäure an der Wildtyp-Position, die sich an der gleichen Position in der Wildtyp-Sequenz des Proteins befindet, ersetzt, wobei die mutierten Sequenzen erhalten werden unter Verwendung einer Parallelsequenzierungsplattform, wobei die Parallelsequenzierungsplattform paralleles Verarbeiten von Nukleinsäure von Krebszellen, das zu Sequenzablesungen führt, und Mapping der Sequenzablesungen mit einer Datenbank mit Referenzgensequenzen anwendet; und
b) Auswählen von mutierten Sequenzen aus denen, die in Schritt a) identifiziert wurden, durch Identifizieren einer HLA-Klasse oder eines HLA-Supertyps des Krebspatienten und danach Auswählen einer Aminosäure für die HLA-Klasse oder den HLA-Supertyp als Aminosäure an der mutierten Position unter Verwendung von FIG. 7;
wobei die Krebsantigene dadurch identifiziert werden, dass Peptide mit den mutierten Sequenzen von Schritt b) synthetisiert werden und hinsichtlich der Aktivierung der von dem Krebspatienten oder von einem HLA-kompatiblen Spender hergestellten Zelllinien von zytotoxischen T-Lymphozyten (CTL-Zelllinien) beurteilt werden, die CTL-Zelllinien durch Inkontaktbringen von einkernigen Zellen von dem Krebspatienten oder von dem HLA-kompatiblen Spender mit Krebszellen von dem Krebspatienten erhalten werden;
- Inkontaktbringen von T-Zellen von dem Krebspatienten oder von einem HLA-kompatiblen Spender mit den Krebsantigenen in vitro, um die Zelllinie von krebsspezifischen zytotoxischen T-Lymphozyten (CTL-Zelllinie) zu stimulieren.

2. Verfahren nach Anspruch 1, wobei das Inkontaktbringen weiterhin das Hinzufügen von autologen CD4⁺-T-Zellen und/oder dendritischen Zellen von dem Krebspatienten oder autologen CD4⁺-T-Zellen und/oder dendritischen Zellen von dem HLA-kompatiblen Spender umfasst.

3. Verfahren nach Anspruch 1, wobei das Vermögen, HLA-Histokompatibilitätsantigene zu binden, unter Verwendung von (einem) computerbasierten Algorithmus/Algorithmen zum Vorhersagen von HLA bindenden Peptiden in silico durchgeführt wird.

4. Verfahren nach Anspruch 1, wobei Peptide mit den ausgewählten Sequenzen von Schritt b) hinsichtlich ihres Vermögens, HLA-Histokompatibilitätsantigene zu binden, beurteilt werden.

5. Verfahren nach Anspruch 4, wobei das Vermögen, HLA-Histokompatibilitätsantigene zu binden, unter Verwendung von (einem) computerbasierten Algorithmus/Algorithmen zum Vorhersagen von HLA bindenden Peptiden in silico durchgeführt wird.

6. Verfahren nach Anspruch 4, wobei das Vermögen, HLA-Histokompatibilitätsantigene zu binden, durch Synthetisieren der Peptide und Testen dieser hinsichtlich der Bindung an antigenpräsentierende Zellen, die HLA-Histokompatibilitätsantigene exprimieren, durchgeführt wird.

7. Verfahren nach Anspruch 1, wobei die Parallelsequenzierungsplattform die Sequenzierungsergebnisse unter Verwendung einer Abdeckung von kleiner als 20x und/oder durch Filtern nicht mit einem BAQ-Algorithmus (BAQ: Base Alignment Quality) filtert.

8. Verfahren nach Anspruch 1, wobei die HLA-Klasse oder der HLA-Supertyp HLA-I ist und die mutierte Aminosäure oder die Wildtyp-Aminosäure ausgewählt ist aus der Gruppe bestehend aus Tyrosin, Asparaginsäure, Glutaminsäure, Leucin, Serin und Threonin, und wobei der Krebspatient das Histokompatibilitätsantigen HLA-A1 exprimiert.

9. Verfahren nach Anspruch 1, wobei die einkernigen Zellen mit CD8⁺-Zellen angereichert werden.

10. Verfahren nach Anspruch 1, wobei das Inkontaktbringen weiterhin das Hinzufügen von autologen CD4⁺-T-Zellen und/oder dendritischen Zellen von dem Krebspatienten oder autologen CD4⁺-T-Zellen und/oder dendritischen Zellen von dem HLA-kompatiblen Spender umfasst.

## Revendications

1. Procédé de fabrication d'une lignée de lymphocytes T cytotoxiques (LTC) spécifiques du cancer pour une utilisation dans le traitement d'un patient atteint d'un cancer, ledit procédé comprenant
- l'identification d'antigènes tumoraux à partir d'un acide nucléique obtenu à partir de cellules cancéreuses issues du patient atteint d'un cancer en utilisant les étapes consistant à
a) obtenir une pluralité de séquences mutantes à partir de l'acide nucléique des cellules cancéreuses issues d'un patient atteint d'un cancer, lesdites séquences mutantes codant pour la totalité ou une partie d'un gène exprimé et les séquences mutantes ayant chacune un acide aminé à une position de mutation qui remplace un acide aminé de type sauvage situé à la même position dans la séquence de type sauvage de la protéine, où lesdites séquences mutantes sont obtenues en employant une plateforme de séquençage parallèle, ladite plateforme de séquençage parallèle utilisant un traitement parallèle dudit acide nucléique des cellules cancéreuses qui produit des lectures de séquences et la cartographie des lectures de séquences dans une banque de données contenant des séquences géniques de référence ; et
b) la sélection de séquences mutantes à partir de celles identifiées à l'étape a) par l'identification d'une classe ou d'un supertype HLA du patient atteint d'un cancer puis la sélection d'un acide aminé pour ladite classe ou ledit supertype HLA en tant qu'acide aminé à une position de mutation en utilisant la FIG. 7 ;
où les antigènes tumoraux sont identifiés en ce que des peptides sont synthétisés qui comprennent lesdites séquences mutantes de l'étape b) et évalués en termes d'activation de lignées de lymphocytes T cytotoxiques (LTC) préparées à partir du patient atteint d'un cancer ou d'un donneur apparié en HLA, lesdites lignées de LTC étant obtenues en mettant en contact des cellules mononucléaires du patient atteint d'un cancer ou du donneur apparié en HLA avec des cellules cancéreuses issues du patient atteint d'un cancer ;
- la mise en contact *in vitro* des lymphocytes T obtenus du patient atteint d'un cancer ou d'un donneur apparié en HLA avec les antigènes tumoraux pour stimuler la lignée de lymphocytes T cytotoxiques (LTC) spécifiques du cancer.

2. Procédé de la revendication 1, où ladite mise en contact inclut en outre l'addition de lymphocytes T CD4⁺ autologues et/ou de cellules dendritiques issus du patient atteint d'un cancer ou de lymphocytes T CD4⁺ autologues et/ou de cellules dendritiques issus du donneur apparié en HLA.

3. Procédé de la revendication 1, où la capacité de liaison aux antigènes d'histocompatibilité HLA est effectuée *in silico* en utilisant un ou des algorithmes informatiques pour prédire quels sont les peptides de liaison aux HLA.

4. Procédé de la revendication 1, où des peptides comprenant les séquences sélectionnées à l'étape b) sont évalués en termes de leur capacité à se lier aux antigènes d'histocompatibilité HLA.

5. Procédé de la revendication 4, où la capacité de liaison aux antigènes d'histocompatibilité HLA est effectuée *in silico* en utilisant un ou des algorithmes informatiques pour prédire quels sont les peptides de liaison aux HLA.

6. Procédé de la revendication 4, où la capacité de liaison aux antigènes d'histocompatibilité HLA est effectuée en synthétisant les peptides et en testant leur capacité à se lier à des cellules présentatrices d'antigènes qui expriment des antigènes d'histocompatibilité HLA.

7. Procédé de la revendication 1, où ladite plateforme de séquençage parallèle filtre les résultats de séquençage en utilisant une profondeur de lecture inférieure à 20 x et/ou en n'appliquant pas un algorithme sur la qualité de l'alignement des bases lors du filtrage.

8. Procédé de la revendication 1, où la classe ou le supertype HLA est HLA-1 et où l'acide aminé mutant ou l'acide aminé de type sauvage est sélectionné dans le groupe consistant en la tyrosine, l'acide aspartique, l'acide glutamique, la leucine, la sérine et la thréonine, et où le patient atteint d'un cancer exprime l'antigène d'histocompatibilité HLA-A1.

9. Procédé de la revendication 1, où lesdites cellules mononucléaires sont enrichies en cellules CD8⁺.

10. Procédé de la revendication 1, où ladite mise en contact inclut en outre l'addition de lymphocytes T CD4⁺ autologues et/ou de cellules dendritiques issus du patient atteint d'un cancer ou de lymphocytes T CD4⁺ autologues et/ou de cellules dendritiques issus du donneur apparié en HLA.
